(19) Europäisches Patentamt
European Patent Office
Office européen des brevets

(11) Numéro de publication : 0 341 231 B1

(12) # FASCICULE DE BREVET EUROPEEN

(45) Date de publication du fascicule du brevet : **19.11.92 Bulletin 92/47**

(51) Int. Cl.[5] : **C07D 233/64,** A61K 31/415

(21) Numéro de dépôt : **89870054.7**

(22) Date de dépôt : **20.04.89**

(54) **1-(1H-imidazol-4-yl)alkyl-benzamides substitués.**

(30) Priorité : **28.04.88 GB 8810067**

(43) Date de publication de la demande : **08.11.89 Bulletin 89/45**

(45) Mention de la délivrance du brevet : **19.11.92 Bulletin 92/47**

(84) Etats contractants désignés : **AT BE CH DE ES FR GB GR IT LI LU NL SE**

(56) Documents cités :
**EP-A- 0 024 829**
**EP-A- 0 058 047**
**EP-A- 0 072 615**
**CHEMICAL ABSTRACTS, vol. 109, no. 9, 29 août 1988, pages 8-9, abstract no. 66276v, Columbus, Ohio, US; J.S. SALONEN et al.:"Metabolism of detomidine in the rat. II. Characterization of metabolites in urine"**

(73) Titulaire : **U C B, S.A.**
**326, Avenue Louise, Bte 7**
**B-1050 Bruxelles (BE)**

(72) Inventeur : **Cossement, Eric**
**105, rue des Echevins**
**B-1050 Bruxelles (BE)**
Inventeur : **Geerts, Jean-Pierre**
**12, Habaru**
**B-6737 Leglise (BE)**
Inventeur : **Gobert, Jean**
**120, rue du Cornet**
**B-1040 Bruxelles (BE)**
Inventeur : **Michel, Philippe**
**54, rue Fr. Couteaux**
**B-1090 Bruxelles (BE)**
Inventeur : **Wülfert, Ernst**
**52, Avenue des Aubépines**
**B-1180 Bruxelles (BE)**

(74) Mandataire : **Dusseldorp, Raymond et al**
**U.C.B. S.A. Département D.T.B. 326, avenue Louise, Bte 7**
**B-1050 Bruxelles (BE)**

EP 0 341 231 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à de nouveaux 1-(1H-imidazol-4-yl)alkyl-benzamides substitués, à leurs sels d'addition d'acides non toxiques pharmaceutiquement acceptables, à des procédés pour les préparer et à leur utilisation dans le domaine thérapeutique.

Elle concerne également les compositions thérapeutiques renfermant lesdits composés.

Selon le brevet européen n° 24.829, on connaît des 4-benzyl-1H-imidazoles dont le groupement benzyle porte sur le noyau phényle divers substituants choisis parmi l'hydrogène et les groupes chloro, bromo, fluoro, méthyle, éthyle, méthoxy, amino, hydroxyle et nitro. Ces composés possèdent des propriétés antihypertensive, antiulcéreuse, diurétique, sédative, analgésique, anti-inflammatoire et tranquillisante. Dans le brevet européen n° 58.047, on décrit des 4-(phénylalkyl)-1H-imidazoles similaires mais dans lesquels le radical alkyle du groupement phénylalkyle comporte 1 à 6 atomes de carbone; dans la plupart des composés, le noyau imidazole est en outre substitué par un radical alkyle contenant 1 à 7 atomes de carbone, un groupe phényle ou un radical benzyle substitué ou non. Ces composés possèdent des activités antithrombotique, antihypertensive, antimicrobienne et antifongique. Dans le brevet européen n° 72.615, on décrit aussi des 4-benzyl-1H-imidazoles similaires mais dans lesquels le groupement benzyle est substitué en position alpha par un radical alkyle. Le groupement benzyle porte sur le noyau phényle divers substituants choisis parmi l'hydrogène, un halogène, les radicaux méthyle, éthyle, hydroxyle, méthoxy et le radical méthylènedioxy entre deux atomes de carbone adjacents. Les essais pharmacologiques décrits dans ce dernier brevet démontrent pour ces composés des propriétés antihypertensive, antithrombotique et diurétique.

Dans la demande de brevet européen n° 269.599 au nom de la demanderesse, on décrit des 1H-imidazoles substitués dont les plus représentatifs sont des 2-hydroxy-3-[1-(1H-imidazol-4-yl)alkyl]benzèneméthanols. Ces 1H-imidazoles possèdent des propriétés anti-ischémiques cardiaque, cérébrale et tissulaire.

Poursuivant ses travaux de recherche dans le domaine, la demanderesse a maintenant synthétisé de nouveaux 1H-imidazoles substitués qui présentent non seulement d'excellentes propriétés anti-ischémiques cardiaque, cérébrale et tissulaire, mais également des propriétés agonistes des récepteurs $\alpha_2$-adrénergiques.

Ces nouveaux composés peuvent donc être utilisés entre autres pour la prévention et le traitement des troubles induits par les ischémies en général. Parmi ces troubles, l'angor est l'expression clinique d'une ischémie myocardique aiguë qui est le résultat d'un déséquilibre momentané entre la demande en oxygène du myocarde et la fourniture en oxygène par la circulation coronaire, déséquilibre pouvant conduire dans les cas graves à l'infarctus du myocarde. C'est pourquoi, ces composés conviennent particulièrement bien pour le traitement de l'angor et de l'infarctus du myocarde. Les propriétés anti-ischémiques au niveau cérébral, permettent l'utilisation de ces composés dans la prévention et le traitement des troubles fonctionnels et neurologiques des accidents vasculaires cérébraux de toute origine (thrombose et infarctus), sans toutefois présenter des propriétés sédatives.

De plus, diverses observations expérimentales telles que des mesures du déplacement de la clonidine marquée au tritium ([$^3$H]clonidine) effectuées avec des préparations de récepteurs $\alpha$-adrénergiques, et des essais pharmacologiques sur des organes isolés, permettent de conclure que les nouveaux composés présentent une puissante activité agoniste des récepteurs $\alpha_2$-adrénergiques. Cette activité est inhibée par l'alpha-yohimbine ce qui classe les composés de l'invention parmi les agonistes des récepteurs $\alpha_2$-adrénergiques. Ces propriétés sont également mises en évidence par la correction de l'élévation des taux de catécholamines plasmatiques ou urinaires due à certaines situations pathologiques reproduites dans des modèles pharmacologiques.

Par voie de conséquence, les nouveaux composés ont des effets thérapeutiques bénéfiques dans le traitement des pathologies induisant ou résultant d'une élévation anormale des taux de catécholamines, telles que phéochromocytome, congestion cardiaque, régulation altérée de la réactivité vasculaire (maladie de Raynaud, migraine ou spasme des artères coronaires), asthme et autres désordres atopiques, glaucome, congestion nasale, maux de tête, tension, stress, anxiété et autres désordres psychiatriques tels que les manies, les dépressions et les troubles de la mémoire (H.J. MOTULSKY et P.A. INSEL, N.Engl.J.Med.307, (1982),18-29; A. DENARO et coll., Acta Psychiatr.Scand.320,(1985, Suppl.72),20-25). Ces mêmes propriétés agonistes des récepteurs $\alpha_2$-adrénergiques autorisent l'utilisation de ces composés dans le traitement des pathologies associées aux hypersécrétions gastriques et intestinales (J.D. DIJOSEPH et coll., Life Sci.35,(1984),1031-1042) ainsi que dans le traitement du syndrome de sevrage au cours des toxicomanies, que celles-ci soient d'origine alcoolique ou résultent de l'abus du tabac et des substances opiacées (G. LAGRUE, Rev.Prat.Médecine générale,1987, n°9 du 23 novembre, 15-17).

Des effets bénéfiques des composés de l'invention, liés à ces propriétés agonistes des récepteurs $\alpha_2$-adrénergiques, peuvent également être attendus dans le traitement des troubles du métabolisme des lipides et des glucides (M.C. HOUSTON et coll., Clin.Res.35,(1987,n°1),17A).

En outre, il a été trouvé que certains de ces composés présentent des activités diurétique, anti-inflammatoire et hypotensive non négligeables.

Les nouveaux composés de la présente invention sont des 1-(1H-imidazol-4-yl)alkyl-benzamides substitués répondant à la formule générale

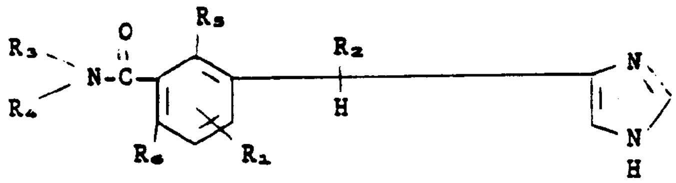

(I)

dans laquelle

$R_1$ et $R_2$, qui peuvent être identiques ou différents, représentent chacun un atome d'hydrogène ou un radical alkyle,

$R_3$ représente un atome d'hydrogène, un radical alkyle ou hydroxyalkyle, un groupe amino ou hydroxyle,

$R_4$ représente un atome d'hydrogène ou un radical alkyle, ou

$R_3$ et $R_4$ pris ensemble avec l'atome d'azote auquel ils sont attachés représentent un radical hétérocyclique choisi parmi les radicaux pyrrolidino, pipéridino et morpholino, et

$R_5$ et $R_5$, qui peuvent être identiques ou différents, représentent chacun un atome d'hydrogène, un groupe hydroxyle, un radical alkyle ou alkoxy,

au moins un des symboles $R_5$ et $R_6$ ayant une signification autre que de l'hydrogène,

tous les radicaux alkyle et alkoxy ayant de 1 à 4 atomes de carbone, ainsi que leurs sels d'addition d'acides non toxiques pharmaceutiquement acceptables.

Lorsque la molécule comporte un atome de carbone asymétrique, les composés de formule I peuvent se présenter soit sous la forme racémique, soit sous forme de l'un ou l'autre énantiomère. Ces diverses formes entrent également dans le cadre de la présente invention.

Les composés préférés conformes à l'invention sont:

- le 2-hydroxy-3-[(1H-imidazol-4-yl)méthyl]-benzamide et son chlorhydrate;
- le 2-hydroxy-3-[1-(1H-imidazol-4-yl)éthyl]-benzamide et son chlorhydrate;
- le 2-hydroxy-3-[(1H-imidazol-4-yl)méthyl]-N-méthylbenzamide et son chlorhydrate;
- le 2,6-dihydroxy-3-[(1H-imidazol-4-yl)méthyl]-benzamide et son chlorhydrate;
- le 2-hydroxy-3-[(1H-imidazol-4-yl)méthyl]-6-méthylbenzamide et son chlorhydrate;
- le 2-hydroxy-5-[(1H-imidazol-4-yl)méthyl]-benzamide;
- le 2-hydroxy-3-[(1H-imidazol-4-yl)méthyl]-benzohydrazide;
- le (+)-2-hydroxy-3-[1-(1H-imidazol-4-yl)éthyl]-benzamide et son chlorhydrate;

La présente invention concerne également les sels d'addition d'acides non toxiques pharmaceutiquement acceptables des 1-(1H-imidazol-4-yl)alkylbenzamides de formule I. Comme exemples d'acides pharmaceutiquement acceptables, on peut citer les acides minéraux comme les acides chlorhydrique, bromhydrique, sulfurique, nitrique, phosphorique, etc. et les acides organiques comme les acides acétique, citrique, tartrique, benzoïque, salicylique, maléique, etc.

Le procédé général de préparation des 1-(1H-imidazol-4-yl)alkyl-benzamides substitués de formule I consiste à faire réagir un 1-(1H-imidazol-4-yl)alkyl-benzoate d'alkyle de formule

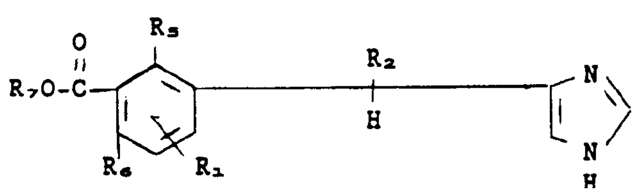

(II)

dans laquelle $R_1$, $R_2$, $R_5$ et $R_5$ ont la signification donnée plus haut et $R_7$ représente un radical alkyle ayant 1 à 4 atomes de carbone, avec un composé azoté de formule

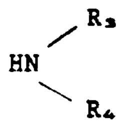

(III)

dans laquelle $R_3$ et $R_4$ ont la signification donnée plus haut.

Cette réaction est généralement effectuée à la pression ordinaire ou à pression plus élevée en autoclave, soit dans un solvant alcoolique comme par exemple le méthanol ou l'éthanol, soit dans un grand excès du composé azoté utilisé comme produit de départ, à une température comprise généralement entre la température ambiante et la température de reflux et éventuellement en présence de méthylate de sodium comme catalyseur.

Suivant une forme de réalisation particulière, réservée à la préparation de 1-(1H-imidazol-4-yl)alkyl-benzamides substitués de formule I dans laquelle $R_5$ et $R_6$ représentent chacun de l'hydrogène, un radical alkyle ou alkoxy ayant 1 à 4 atomes de carbone, au moins un des symboles $R_5$ et $R_5$ ayant une signification autre que de l'hydrogène, on fait réagir un acide 1-(1H-imidazol-4-yl)alkyl-benzoïque de formule

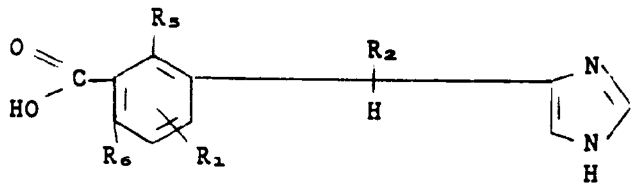

(IV)

dans laquelle $R_1$ et $R_2$ représentent chacun un atome d'hydrogène ou un radical alkyle ayant 1 à 4 atomes de carbone et $R_5$ et $R_5$ ont la signification donnée ci-dessus, avec un composé azoté de formule

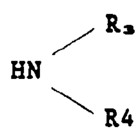

(III)

dans laquelle $R_3$ et $R_4$ ont la signification donnée plus haut.

Pour réaliser cette réaction, l'acide de départ de formule IV est au préalable activé, de manière connue en soi, au moyen d'un réactif conventionnel tel que par exemple un halogénoformiate d'alkyle, de préférence le chloroformiate d'éthyle. Cette réaction est généralement effectuée à une température voisine de 0°C, dans un solvant inerte tel que par exemple le dichlorométhane ou l'acétonitrile, et en présence d'une base auxiliaire telle que par exemple la triéthylamine.

Suivant encore une autre forme de réalisation, réservée à la préparation de 1-(1H-imidazol-4-yl)alkyl-benzamides substitués de formule I, dans laquelle $R_1$, $R_3$ et $R_4$ représentent de l'hydrogène, $R_5$ représente un groupe hydroxyle et $R_6$ représente de l'hydrogène ou un radical alkyle ayant 1 à 4 atomes de carbone, on hydrolyse en milieu acide un 2-hydroxy-3-[1-(1H-imidazol-4-yl)alkyl]-benzonitrile de formule

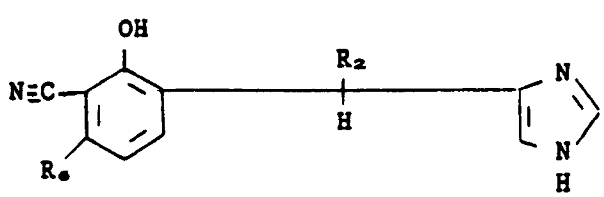

(V)

dans laquelle $R_2$ et $R_5$ représentent chacun un atome d'hydrogène ou un radical alkyle ayant 1 à 4 atomes de carbone.

Cette hydrolyse est généralement effectuée au moyen d'une solution aqueuse d'acide sulfurique d'environ 80% en volume, à une température comprise entre 60 et 75°C pendant quelques heures.

Selon une variante, cette hydrolyse peut aussi être effectuée dans de l'alcool méthylique anhydre auquel

on a ajouté une trace d'eau et dans lequel on fait passer un courant d'acide chlorhydrique gazeux. L'imidate intermédiaire qui se forme in situ n'est pas isolé et est directement transformé en amide par chauffage.

Les sels d'addition d'acides non toxiques pharmaceutiquement acceptables peuvent être préparés à partir des 1-(1H-imidazol-4-yl)alkyl-benzamides de formule I par des méthodes connues en soi.

Les composés de formule I dans laquelle $R_2$ est un radical alkyle et qui, de ce fait, se présentent sous la forme d'un mélange racémique, peuvent être séparés en leurs énantiomères optiques par des méthodes conventionnelles, soit par cristallisation fractionnée des sels diastéréoisomères obtenus par addition au mélange racémique d'un acide optiquement actif, soit par chromatographie du mélange racémique sur un support chiral tel que par exemple une silice sur laquelle est greffée de manière covalente de l'albumine sérique bovine (BSA) ou une phase contenant de l'alpha-glycoprotéine ou de la bêta-cyclodextrine. Plusieurs passages successifs sur la colonne chromatographique chirale sont parfois nécessaires pour améliorer la séparation des énantiomères.

Les 1-(1H-imidazol-4-yl)alkyl-benzoates d'alkyle de départ de formule II peuvent être préparés par l'un ou l'autre des procédés suivants:

(a) on estérifie selon les méthodes classiques un acide 1-(1H-imidazol-4-yl)alkyl-benzoïque de formule IV dans laquelle $R_1$, $R_2$, $R_5$ et $R_6$ ont la signification donnée plus haut, avec un alcanol de formule $R_7OH$ dans laquelle $R_7$ représente un radical alkyle ayant 1 à 4 atomes de carbone;

(b) lorsque $R_6$ représente un radical alkoxy en $C_1$-$C_4$ et $R_6$ un atome d'hydrogène, un radical alkyle en $C_1$-$C_4$ ou alkoxy en $C_1$-$C_4$, on peut également utiliser un procédé en plusieurs stades:

(1) réaction en présence d'une base, dans l'acétone à reflux, d'un 2-hydroxybenzoate d'alkyle de formule VI, convenablement substitué, avec un 2,3-dichloropropène de formule VII pour obtenir un 2-(2-chloro-2-propényloxy)-benzoate d'alkyle de formule VIII, selon l'équation

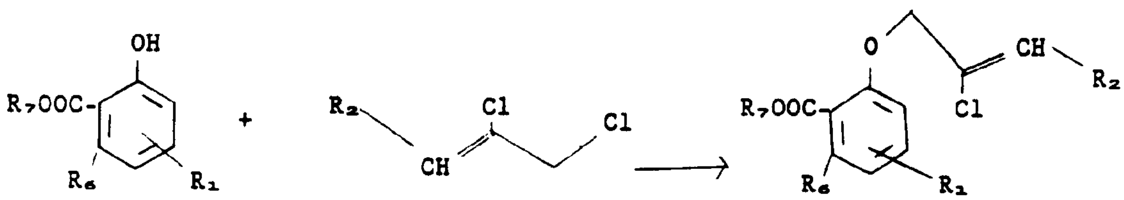

(VI) (VII) (VIII)

(2) chauffage à une température voisine de 260°C du 2-(2-chloro-2-propényloxy)-benzoate d'alkyle de formule VIII, ce qui conduit par une transformation de Claisen au 3-(2-chloro-2-propényl)-2-hydroxybenzoate d'alkyle de formule IX, selon l'équation

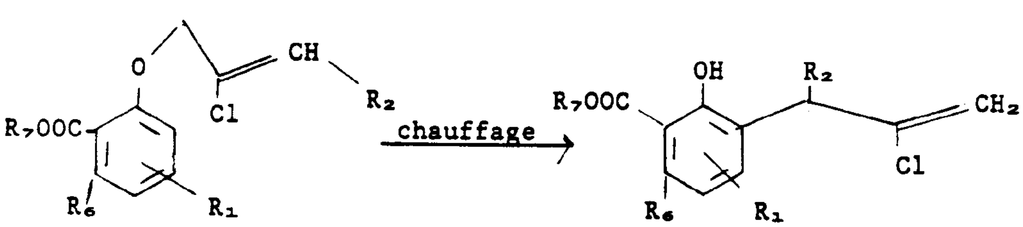


(VIII) (IX)

(3) alkylation du 3-(2-chloro-2-propényl)-2-hydroxybenzoate d'alkyle de formule IX par un halogénure de $R_8$, selon l'équation

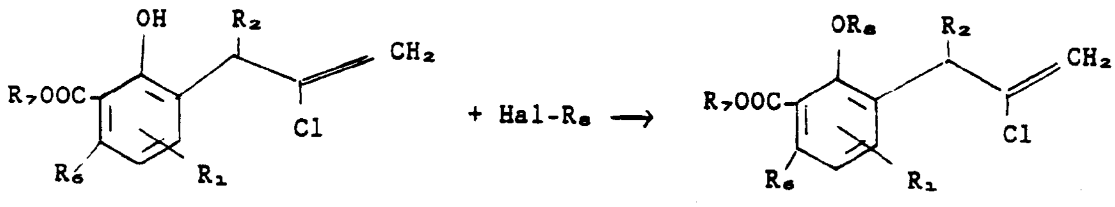


(IX) (X)

(4) oxydation par l'acide m-chloroperbenzoïque (mCPBA) dans le chloroforme à la température de reflux pendant plusieurs heures, du 3-(2-chloro-2-propényl)-2-($R_8$-oxy)-benzoate d'alkyle de formule X, selon l'équation

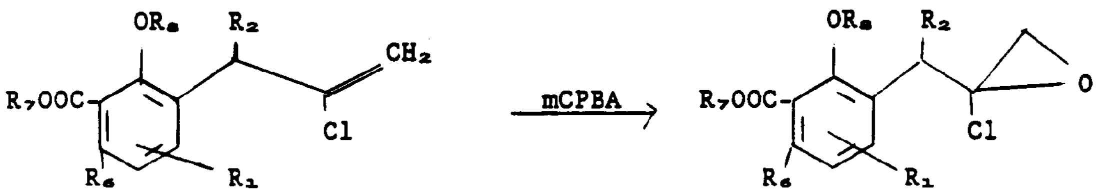


(X) (XI)

(5) réaction en présence d'une base et à une température voisine de 60°C, de l'époxy-ester de formule XI avec l'acétate de formamidine, ce qui conduit au 1-(1H-imidazol-4-yl)alkyl-benzoate d'alkyle de formule II, selon l'équation

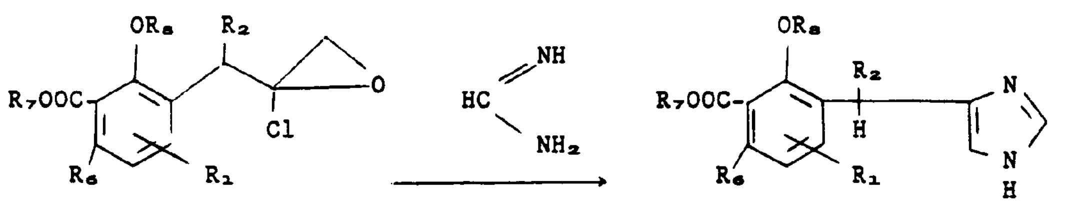

(XI)

(II) avec $R_5$ = $OR_8$ = $C_1$-$C_4$-alkoxy,
$R_6$ = H, $C_1$-$C_4$-alkyle ou
$C_1$-$C_4$-alkoxy.

dans ces formules, $R_1$ et $R_2$ représentent chacun un atome d'hydrogène ou un radical alkyle en $C_1$-$C_4$, $R_6$ a la signification donnée ci-dessus, $R_7$ représente un radical alkyle ayant 1 à 4 atomes de carbone, de préférence le radical méthyle ou éthyle, $R_8$ représente un radical alkyle ayant 1 à 4 atomes de carbone et Hal est un atome d'halogène, de préférence le chlore ou le brome.

(c) lorsque $R_8$ représente un groupe hydroxyle et $R_8$ un atome d'hydrogène, un radical alkyle en $C_1$-$C_4$ ou alkoxy en $C_1$-$C_4$, on opère suivant une variante du procédé (b) ci-dessus; selon cette variante, on se borne à exécuter uniquement les stades (1), (2), (4) et (5) du procédé (b) en omettant l'alkylation avec un halogénure de $R_8$ du stade (3). On soumet donc directement le composé de formule IX qui est obtenu au stade (2) à l'oxydation par l'acide m-chloroperbenzoïque.

Ces mêmes esters peuvent également être préparés à partir d'un 2-oxo-cyclohexanecarboxylate d'alkyle et d'un 4-(1-chloroalkyl)-1H-imidazole par le procédé en plusieurs stades décrit dans la demande de brevet européen n° 269.599 au nom de la demanderesse (voir exemple 4.13. ci-après)

(d) on fait réagir un benzoate d'alkyle de formule XII avec un 1H-imidazole-4-méthanol de formule XIII selon l'équation

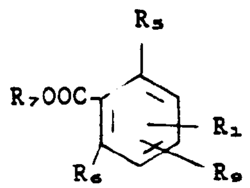

+

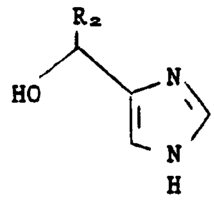

(XII) (XIII)

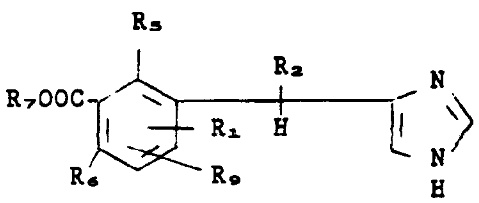

(II) éventuellement halogéné en $R_9$.

dans ces formules $R_1$, $R_2$, $R_5$ et $R_6$ ont la signification donnée plus haut, $R_7$ représente un radical alkyle ayant 1 à 4 atomes de carbone, et $R_9$ représente un atome d'hydrogène ou d'halogène, tel que le brome. Cette réaction de Friedel et Crafts est généralement effectuée dans un acide inorganique tel que l'acide sulfurique concentré ou l'acide polyphosphorique ou un acide organique tel que l'acide formique, ou dans un mélange des acides précités, pendant plusieurs heures à une température comprise entre 20 et 100°C. Ce procédé ne fournit pas toujours qu'un seul composé. En effet, on obtient parfois un mélange des isomères de position 3-[1-(1H-imidazol-4-yl)alkyl]-benzoate d'alkyle et 5-[1-(1H-imidazol-4-yl)alkyl]-benzoate d'alkyle à partir duquel les deux isomères peuvent être séparés et purifiés par chromatographie.
L'expérience montre cependant que la séparation chromatographique est parfois plus facilement réalisable au niveau des acides qu'à celui des esters. C'est pourquoi, si nécessaire, le mélange des esters isomères obtenu est d'abord soumis à une hydrolyse qui conduit au mélange des acides isomères correspondants, lesquels sont alors séparés par chromatographie. Ces acides sont ensuite réestérifiés pour fournir les esters de formule II désirés. Lorsque dans les 1-[(1H-imidazol-4-yl)alkyl]-benzoates d'alkyle ainsi obtenus $R_9$ représente un atome d'halogène, on élimine cet atome d'halogène au cours d'une étape supplémentaire d'hydrogénolyse afin d'obtenir les composés correspondants de formule II.
(e) lorsque $R_1$ représente un atome d'hydrogène, $R_5$ un groupe hydroxyle et $R_6$ de l'hydrogène ou un radical alkyle ayant 1 à 4 atomes de carbone, on peut aussi utiliser un procédé en plusieurs stades:

(1) réaction en présence d'éthylate de sodium d'un 4-(1-chloroalkyl)-1H-imidazole de formule XIV avec deux équivalents d'un 4-hydroxy-3-oxo-butanoate d'alkyle, de préférence d'éthyle, de formule XV et dont la fonction hydroxyle est protégée, pour obtenir un 4-hydroxy-2-[1-(1H-imidazol-4-yl)alkyl]-3-oxo-butanoate d'alkyle de formule XVI selon l'équation

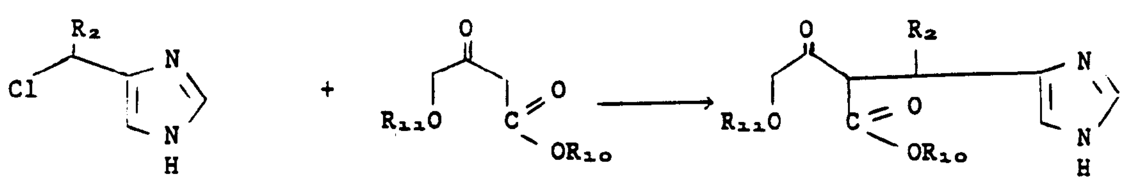

(XIV) (XV) (XVI)

(2) réduction du bêta-cétoester de formule XVI en bêta-hydroxyester de formule XVII au moyen de borohydrure de sodium selon l'équation

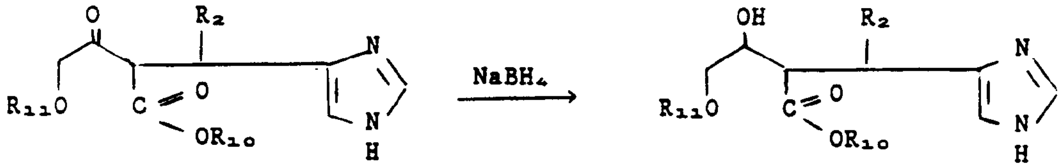


(XVI) (XVII)

(3) déprotection et cyclisation simultanée du bêta-hydroxyester de formule XVII par des méthodes connues en soi pour obtenir une 4-hydroxy-3-[1-(1H-imidazol-4-yl)alkyl]-dihydro-2(3H)-furanone de formule XVIII selon l'équation

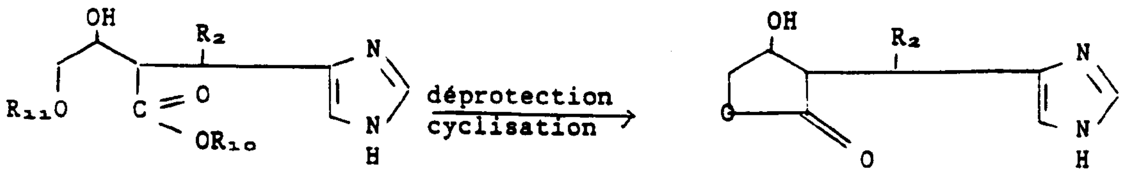


(XVII) (XVIII)

(4) déshydratation thermique de la 4-hydroxy-3-[1-(1H-imidazol-4-yl)alkyl]-dihydro-2(3H)-furanone de formule XVIII par chauffage à température élevée et sous vide, pour obtenir une 3-[1-(1H-imidazol-4-yl)alkyl]-2(5H)-furanone de formule XIX selon l'équation

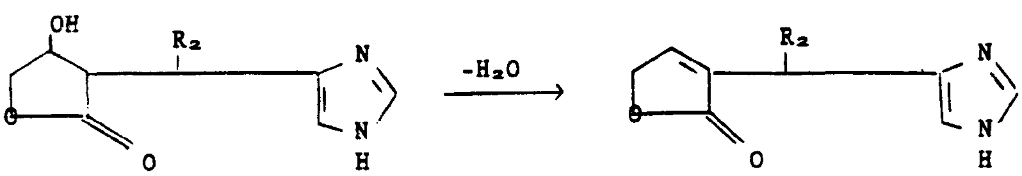


(XVIII) (XIX)

Cette déhydratation peut aussi être effectuée par chauffage dans un solvant inerte à point d'ébullition élevé, comme par exemple l'éthylène glycol.

(5) réaction de cycloaddition de la 3-[1-(1H-imidazol-4-yl)alkyl]-2(5H)-furanone de formule XIX avec un acrylate d'alkyle de formule XX, ce qui conduit au 1-(1H-imidazol-4-yl)alkyl-benzoate d'alkyle de formule II selon l'équation

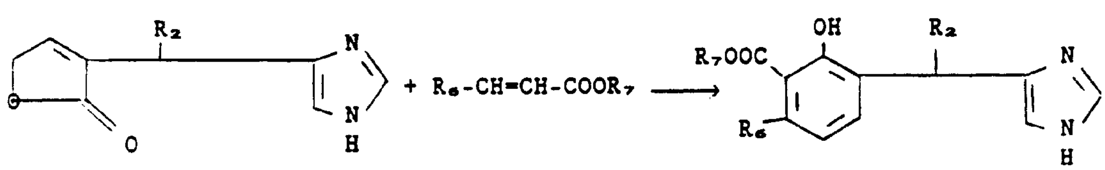

(XIX) (XX) (II) avec $R_1$ = H
$R_5$ = OH
$R_6$ = H ou $C_1$-$C_4$-alkyle.

Cette réaction de Diels-Alder est tout d'abord effectuée en présence de triéthylamine et de triméthylchlorosilane qui transforme in situ la 2-(5H)-furanone de formule XIX en 2-triméthylsilyloxyfuranne (diène) correspondant. Lorsque la réaction de cet intermédiaire silylé avec l'acrylate d'alkyle est terminée,

le produit d'addition primaire (un oxanorbornène) est hydrolysé et aromatisé en hydroxybenzoate de formule II par chauffage pendant quelques minutes dans de l'acide chlorhydrique ou bromhydrique concentré.

Dans ces formules, $R_2$ représente un atome d'hydrogène ou un radical alkyle ayant 1 à 4 atomes de carbone, $R_6$ a la signification donnée ci-dessus, $R_7$ et $R_{10}$ représentent chacun un radical alkyle ayant 1 à 4 atomes de carbone, de préférence le radical méthyle ou éthyle et $R_{11}$ représente un groupe protecteur classique de la fonction hydroxyle choisi parmi les radicaux méthyle, tert-butyle, benzyle et benzoyle, de préférence le radical benzyle.

Les 4-(1-chloroalkyl)-1H-imidazoles de formule XIV peuvent être préparés à partir des 1H-imidazole-4-méthanols correspondants par chloruration selon des méthodes connues en soi (J.L. KELLEY et al., J.Med.Chem.20,(1977),721-723).

Les 4-hydroxy-3-oxo-butanoates d'alkyle de formule XV dont la fonction hydroxyle est protégée par le radical $R_{11}$ peuvent être préparés à partir des 4-chloro-3-oxo-butanoates d'éthyle correspondants selon la méthode décrite par T. MEUL et al., Chimia,41,(1987),73-76.

Les acides 1-(1H-imidazol-4-yl)alkyl-benzoïques de formule IV utilisés comme produits de départ, soit pour la préparation des 1-(1H-imidazol-4-yl)alkyl-benzoates d'alkyle de formule II selon le procédé (a) ci-dessus, soit pour la préparation des composés de formule I, peuvent être obtenus par l'un ou l'autre des procédés suivants:

(1) oxydation des 1-(1H-imidazol-4-yl)alkyl-benzèneméthanols correspondants de formule XXI, selon l'équation

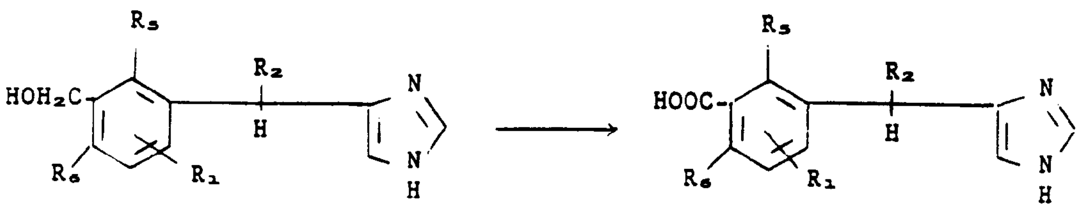

(XXI) (IV)

dans ces formules, $R_1$, $R_2$, $R_5$ et $R_6$ ont la signification donnée plus haut.

Cette réaction d'oxydation est effectuée par chauffage de l'alcool de départ vers 170-190°C, pendant plusieurs heures, dans de l'hydroxyde de potassium en fusion. L'acide est isolé après dissolution du mélange réactionnel dans de l'eau et acidification de la solution aqueuse du sel de potassium de l'acide.

La préparation des alcools de formule XXI utilisés comme matières premières dans cette réaction est décrite dans la demande de brevet européen n° 269.599 au nom de la demanderesse.

(2) hydrolyse selon les méthodes conventionnelles des esters correspondants préparés par l'un des procédés (b), (c), (d) ou (e) décrits plus haut.

Lorsqu'on part d'un mélange d'esters isomères, préparé selon le procédé (d), le mélange des acides isomères obtenus est soumis à une chromatographie pour séparer les acides individuels.

Les 2-hydroxy-3-[1-(1H-imidazol-4-yl)alkyl]-benzonitriles de départ de formule V peuvent être préparés en commençant par les stades (1) à (4) du procédé (e) de préparation des esters de formule II décrit ci-dessus pour préparer la 3-[1-(1H-imidazol-4-yl)alkyl]-2(5H)-furanone de formule XIX, que l'on fait réagir ensuite avec un acrylonitrile de formule XXII, selon l'équation

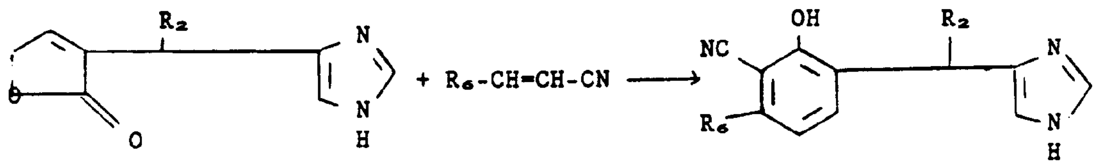

(XIX) (XXII) (V)

Les conditions dans lesquelles cette réaction de cycloaddition est réalisée sont les mêmes que celles qui sont décrites plus haut pour le stade (5) du procédé (e).

Dans ces formules, $R_2$ et $R_6$ représentent chacun un atome d'hydrogène ou un radical alkyle ayant 1 à 4 atomes de carbone.

Comme il a été indiqué plus haut, les 1-(1H-imidazol-4-yl)alkyl-benzamides substitués de formule I, ainsi que leurs sels d'addition d'acides non toxiques pharmaceutiquement acceptables, sont doués de propriétés pharmacologiques de valeur; en particulier, il a été trouvé qu'ils possèdent d'excellentes propriétés anti-ischémiques cardiaque et cérébrale ainsi que des propriétés agonistes des récepteurs $\alpha_2$-adrénergiques.

Les essais pharmacologiques décrits ci-après mettent en évidence ces diverses propriétés.

Les produits suivants de la présente invention ont été soumis à des essais pharmacologiques:

- chlorhydrate de 2-hydroxy-3-[(1H-imidazol-4-yl)méthyl]-benzamide (produit A),
- chlorhydrate de 2-hydroxy-3-[1-(1H-imidazol-4-yl)éthyl]-benzamide (produit B),
- chlorhydrate de 2-hydroxy-3-[(1H-imidazol-4-yl)méthyl]-N-méthylbenzamide (produit C),
- 2-hydroxy-3-[(1H-imidazol-4-yl)méthyl]-benzohydrazide (produit D),
- chlorhydrate de 2-hydroxy-N-(2-hydroxyéthyl)-3-[(1H-imidazol-4-yl)méthyl]-benzamide (produit E),
- chlorhydrate de 3-[(1H-imidazol-4-yl)méthyl]-2-méthoxybenzamide (produit F),
- 2-hydroxy-5-[(1H-imidazol-4-yl)méthyl]-benzamide (produit G),
- 2-hydroxy-3-[(1H-imidazol-4-yl)méthyl]-N,N-diméthylbenzamide (produit H),
- chlorhydrate de 2,6-dihydroxy-3-[(1H-imidazol-4-yl)méthyl]-benzamide (produit I),
- 5-tert-butyl-2-hydroxy-3-[(1H-imidazol-4-yl)méthyl]-benzamide (produit J),
- chlorhydrate de 2-hydroxy-3-[(1H-imidazol-4-yl)méthyl]-6-méthylbenzamide (produit K),
- 2-hydroxy-3-[(1H-imidazol-4-yl)méthyl]-4-méthylbenzamide (produit L),
- chlorhydrate de N,2-dihydroxy-3-[(1H-imidazol-4-yl)méthyl]-benzamide (produit M),
- chlorhydrate de 2,6-dihydroxy-3-[1-(1H-imidazol-4-yl)éthyl]-benzamide (produit N),
- chlorhydrate de 6-hydroxy-3-[(1H-imidazol-4-yl)méthyl]-2-méthylbenzamide (produit O),
- chlorhydrate de (+)-2-hydroxy-3-[1-(1H-imidazol-4-yl)éthyl]-benzamide (produit P).

1. Activité anti-ischémique cardiaque.

a) Modèle d'insuffisance coronaire aigüe chez le chien éveillé.

Chez le chien appareillé (manchette pneumatique autour de l'artère inférieure coronaire descendante et électrodes intracardiaques) et éveillé, une occlusion de l'artère coronaire, d'une durée de 6 minutes, est réalisée au moyen d'une manchette pneumatique. En raison de la réduction de l'apport d'oxygène qui en résulte, cette occlusion provoque une ischémie myocardique qui se traduit sur l'électrocardiogramme par une élévation, reproductible et quantifiable, du segment ST. L'action anti-ischémique d'un composé se manifeste par une diminution de l'élévation du segment ST (P.R. MAROKO et E. BRAUNWALD, Circulation,53,(1976,Suppl.I),162-168; S.E. EPSTEIN et al., Circulation,53,(1976,Suppl.I),191-197).

Le tableau I montre pour les produits soumis à l'essai la dose ($DE_{20}$ en µmole/kg) qui, administrée par voie intraveineuse à des groupes de 10 animaux, produit une diminution moyenne d'au moins 20% de l'élévation du segment ST sur l'ensemble des animaux. Comme substance de référence, on utilise le 1-(isopropylamino)-3-(1-naphtyloxy)-2-propanol (ou propranolol).

Tableau I

| Produits | $DE_{20}$ (en µmole/kg) |
|---|---|
| A | 0,03 |
| B | 0,02 |
| C | 0,3 |
| D | 3 |
| E | < 3 |
| F | < 3 |
| G | < 1 |
| I | 0,32 |
| K | 0,32 |
| propranolol | 2 |

De ce tableau, il ressort que les composés de l'invention présentent une activité anti-ischémique remarquable.

b) Epreuve d'effort sur tapis roulant.

Dans cet essai, on utilise un groupe d'au moins 4 chiens appareillés (électrodes intracardiaques) qui présentent au niveau d'une artère coronaire une sténose organique. Celle-ci provoque le déséquilibre entre la demande et l'apport d'oxygène lorsqu'on demande à l'animal de fournir un effort. Ce déséquilibre se traduit sur l'électrocardiogramme par l'augmentation du segment ST.
Dans l'épreuve, le chien court à la vitesse de 12 km/h sur un tapis roulant présentant une pente de 15 degrés. Cet effort maximal est demandé pendant 1 minute. Pendant l'épreuve, on enregistre l'augmentation du segment ST et l'augmentation naturelle de la fréquence cardiaque. L'expérience est répétée au moins 4 fois et la moyenne des valeurs obtenues est prise comme référence (100%) pour le groupe d'animaux. L'animal est ensuite mis au repos pendant un minimum de 24 heures avant d'être soumis à une nouvelle épreuve d'effort sous influence du composé à étudier.
Le composé à étudier est administré lentement (en 1 minute) par voie intraveineuse 5 minutes avant la nouvelle épreuve d'effort. Au cours de celle-ci, on enregistre les variations des mêmes paramètres.
Dans le tableau II, on donne pour la dose indiquée (µmole/kg) la diminution moyenne observée pour le segment ST (en %) et pour la fréquence cardiaque (en battements/min.) par rapport aux valeurs de référence obtenues dans l'épreuve initiale.

Tableau II

Effort maximal à 12 km/h pendant 1 minute.

| Produits | doses (µmole/kg) | diminution du segment ST (en %) | diminution de la fréquence cardiaque (en battements/min.) |
|---|---|---|---|
| A | 0,018 | 74 | 3 |
| C | 0,32 | 50 | 1 |
| propranolol | 1,0 | 74 | 35 |

De ce tableau, il ressort que les composés de l'invention ont une bonne activité anti-ischémique démontrée par une forte diminution du segment ST, ce qui est aussi constaté avec le propranolol, mais seulement à une dose beaucoup plus élevée. De plus, contrairement au propranolol, qui provoque en même temps une forte diminution du rythme cardiaque pendant l'épreuve, ce qui n'est pas souhaitable et qui est nuisible au maintien de l'effort, les composés de l'invention ne s'opposent pas à l'augmentation naturelle de la fréquence cardiaque pendant l'épreuve. Ils permettent donc une adaptation correcte de la fréquence cardiaque à l'effort tout en s'opposant à l'ischémie.

2. Activité anti-ischémique cérébrale.

a) Ischémie cérébrale globale et permanente chez le rat.

Des rats Wistar mâles (200 à 250 g) sont anesthésiés par inhalation d'halothane (1 à 5%) contenu dans un mélange $N_2O$-$O_2$ (70:30). Les deux artères carotides communes sont ligaturées simultanément à proximité du carrefour entre carotide interne et carotide externe selon la technique décrite par M. LE PONCIN-LAFITTE et coll., J.Pharmacol. (Paris),14,(1983),99-102.
Le produit à tester est administré par voie intrapéritonéale une première fois 30 minutes avant les ligatures et ensuite 30 minutes et 270 minutes après celles-ci.
Le lendemain et le surlendemain, on apprécie le déficit neurologique des animaux survivants par la méthode décrite par C. CAPDEVILLE et coll., J.Pharmacol. (Paris),15,(1984),231-237, et par B. KOLB et coll., Neurobehav.Toxicol.Teratol.7,(1985),71-78. Les fonctions sensori-motrices prises en considération lors de cette appréciation sont la motilité spontanée, le réflexe d'agrippement, les réactions de placement visuelles et par perte d'appui, le réflexe de flexion des pattes, le réflexe de redressement et le test de pendaison par la queue. Le score maximal possible pour un animal non soumis à une ischémie est de 17.
Dans le tableau III, on donne pour le composé A, administré par voie intrapéritonéale à la dose de 0,76 µg/kg (3,2 nmole) la moyenne des scores neurologiques déterminés sur l'ensemble des animaux survivants du groupe témoin et du groupe traité, enregistrés 2 jours après les ligatures. La signification statistique (P) de la différence observée entre ces moyennes est évaluée par le test de Mann-Whitney.

Tableau III

Scores neurologiques après 2 jours.

| | |
|---|---|
| témoin (n = 16) | 12 |
| A (n = 15) | 15 |
| (P) | (0,005) |

n = nombre d'animaux survivants.

Il ressort de ce tableau que le composé A, à très faible dose, atténue significativement chez les animaux traités le déficit neurologique provoqué par l'ischémie.

b) Ischémie cérébrale multifocale unilatérale chez le rat.

Chez des rats mâles Sprague-Dawley SPF éveillés, âgés de 8 à 9 semaines, on provoque une ischémie cérébrale unilatérale permanente (ou embolisation) par introduction dans le flux carotidien droit de 2000 microsphères (fourni par 3M, St. Paul, Etats-Unis; diamètre 58±2 µm) (A.M. BRALET et coll., Stroke,10,(1979),34-38; M. LE PONCIN-LAFITTE et coll., Pathol.Biol. (Paris),30,(1982),289-293) après la ligature permanente de l'artère ptérygopalatine droite (Y. KIYOTA et coll., Pharmacol.Biochem. Behav.24,(1986),687-692).
Le composé à étudier est administré une première fois 30 minutes avant et une seconde fois 30 minutes après l'embolisation, tandis que les animaux témoins ne reçoivent que du sérum physiologique. Ensuite, on laisse les animaux au repos. Après 6 jours de récupération on mesure chez les animaux survivants:

1) le déficit neurologique résiduel au moyen du test de la posture et de la démarche des animaux (Test A, score maximum: 4 points). Ce test évalue
   a) le positionnement anormal des pattes postérieures (S. IRWIN, Psychopharmacologia (Berlin),13,(1968),222-257);
   b) l'inclinaison contra-latérale du corps lors de la locomotion;
   c) la flexion longitudinale homolatérale du corps, et
   d) la démarche anormale (B. KOLB et coll., loc.cit.).

2) les fonctions sensori-motrices (motilité spontanée, réflexe d'agrippement, réactions de placement visuelles et par perte d'appui (Test B, score maximum: 10 points); (C. CAPDEVILLE et coll., loc.cit.).

3) la réponse sensori-motrice latéralisée (côté contra-latéral). (Test C, score maximum: 3 points). Elle est évaluée en combinant les mesures du réflexe de placement visuel, du réflexe d'orientation de la tête vers un stimulus sensoriel latéral et du réflexe cutané plantaire (C. CAPDEVILLE et coll., loc.cit.; J.F. MARSHALL et coll., Science (Washington),174,(1971),523-525).

4) l'extinction tactile du côté gauche (test D, score maximum: 300 points). Contrairement aux autres tests

cités ci-dessus pour lesquels le déficit est d'autant plus atténué que le score est plus élevé, le déficit est ici d'autant plus prononcé que le score est plus proche de 300 (T. SCHALLERT et coll., Pharmacol.Biochem. Behav.16,(1982),455-462).

Le 7e jour de récupération on mesure encore l'oedème présent dans diverses structures cérébrales ipsilatérales (M. LE PONCIN-LAFITTE et coll., loc.cit.).

Dans le tableau IV on donne pour le composé A, administré par voie intrapéritonéale à la dose de 0,76 µg/kg (3,2 nmoles/kg), les résultats obtenus aux tests A à D, c'est-à-dire la moyenne des scores neurologiques déterminés sur l'ensemble des animaux survivants du groupe témoin et du groupe traité, après 6 jours de récupération. On y a indiqué également la variation moyenne (en g) du poids corporel, mesurée au 7e jour de récupération.

La signification statistique (P) de la différence entre les valeurs moyennes calculées pour les animaux témoins et les animaux traités est évaluée par le test de Mann-Whitney.

Ce tableau montre que le composé A atténue significativement le déficit neurologique et comportemental induit par l'ischémie et améliore l'évolution pondérale des animaux traités.

Tableau IV

Scores neurologiques moyens 6 jours après l'embolisation.

| groupe | n(*) | test A | test B | test C | test D | Variation du poids (en g) |
|---|---|---|---|---|---|---|
| témoin | 18 | 1,0 | 7,3 | 1,0 | 243 | -5,7 |
| traité | 17 | 2,0 | 8,0 | 2,0 | 96 | +0,8 |
| (P) | | (< 0,01) | (< 0,05) | (< 0,005) | (< 0,05) | (< 0,05) |

(*): n indique le nombre d'animaux survivants dans chaque groupe.

Dans le tableau V on indique la quantité d'eau (moyenne des pourcentages) retenue dans diverses structures cérébrales ipsilatérales chez les animaux témoins et chez les animaux traités survivants au 7e jour après l'embolisation.

Les résultats obtenus montrent que le traitement par le composé A diminue significativement l'oedème ipsilatéral dans les différentes structures cérébrales étudiées.

Tableau V.

Quantité d'eau dans diverses structures cérébrales ipsilatérales (% moyens)

| groupe | n(*) | Hippocampe | Corps strié | Diencephale | Cortex |
|---|---|---|---|---|---|
| témoin | 18 | 80,20 | 81,57 | 77,04 | 81,33 |
| traité | 17 | 79,79 | 80,05 | 76,32 | 80,36 |
| (P) | | (< 0,05) | (< 0,005) | (< 0,05) | (< 0,01) |

(*): n indique le nombre d'animaux survivants dans chaque groupe.

3. Propriété agoniste $\alpha_2$-adrénergique.

a) Fixation compétitive avec un radioligand.

Les expériences de fixation compétitive ont pour but de mesurer l'affinité qu'ont les composés de l'invention pour les récepteurs $\alpha_2$-adrénergiques. Ces expériences classiques mettent en jeu la compétition de la fixation sur les récepteurs $\alpha_2$-adrénergiques d'une part, du composé à étudier et d'autre part, d'un radioligand qui, dans

ce cas particulier des récepteurs $\alpha_2$-adrénergiques, est la [$^3$H]clonidine connue pour être un agoniste $\alpha_2$-adrénergique sélectif.
La méthode utilisée est celle de D.C. U'PRICHARD et coll., Mol. Pharmacol.13,(1977),454-473.
Les courbes de déplacement de la fixation de la [$^3$H]clonidine ont été déterminées avec neuf concentrations du composé A, comprises entre $10^{-4}$ et $10^{-10}$ mole/l et trois préparations membranaires différentes de cerveau de rat. Les échantillons ont été incubés pendant 30 minutes, puis filtrés sous vide sur filtre Whatman GF/B. Les filtres sont lavés trois fois avec 5 ml de tampon Tris-HCl (pH 7,5 à 0°C) puis séchés pendant une minute. La radioactivité est mesurée dans un milieu Econofluor (-NEN Corp.). La [$^3$H]clonidine (25,5 Ci/mmole) utilisée est fournie par Amersham.
L'affinité du composé A pour les récepteurs $\alpha_2$-adrénergiques a été calculée à partir des courbes de déplacement de la [$^3$H]clonidine. Elle est exprimée par la concentration ($IC_{50}$ en mole/l) du composé A nécessaire pour obtenir une inhibition de 50% de la fixation du radioligand sur les récepteurs. Les résultats obtenus montrent que le composé A présente une importante affinité pour les récepteurs $\alpha_2$-adrénergiques:

$$IC_{50} = 8,90 \pm 0,72 \times 10^{-9} \text{ mole/l}$$

b) Stimulation de l'oreillette isolée de cobaye.

La libération de noradrénaline au niveau des terminaisons nerveuses est régulée par un mécanisme de rétrocontrôle par les récepteurs $\alpha_2$-adrénergiques pré-synaptiques. Ce mécanisme a été démontré sur l'oreillette de cobaye par M.J. RAND et coll., "Central action drugs in blood pressure regulation", 1975, 94-132. Ed. D.S. DAVIES, J.L. REID, Pitman, Londres.
La stimulation électrique de l'oreillette isolée de cobaye induit une libération de noradrénaline qui se traduit par une augmentation de la fréquence de battement du coeur (tachycardie). Cette tachycardie est inhibée par un $\alpha_2$-agoniste, tel que par exemple la clonidine, dans une proportion qui dépend de la dose d'agoniste utilisée. L'action de l'$\alpha_2$-agoniste peut être limitée en présence d'un antagoniste $\alpha_2$-spécifique, tel que l'alpha-yohimbine. L'activité in vitro des composés de l'invention vis-à-vis des récepteurs $\alpha_2$-adrénergiques pré-synaptiques a été étudiée sur l'oreillette isolée de cobaye stimulée électriquement selon la méthode décrite par I.C. MEDGETT et coll., Naunyn-Schmiedeberg's Arch. Pharmacol.,304,(1978),215-221. Le composé à étudier est testé à des concentrations croissantes comprises entre $10^{-10}$ et $10^{-3}$ mole/l. On détermine la concentration ($IC_{30}$ en mole/l) qui provoque une réduction de 30% de la tachycardie maximale obtenue initialement lors de la stimulation électrique de l'oreillette en l'absence du composé à tester.
Le tableau VI montre les concentrations $IC_{30}$ (en mole/l) obtenues pour les composés de l'invention ainsi que pour la clonidine.

Tableau VI.

Réduction de la tachycardie.

| produits | n(*) | $IC_{30}$ (en mole/l) |
|---|---|---|
| A | 7 | $9 \times 10^{-10}$ |
| B | 6 | $7,5 \times 10^{-10}$ |
| C | 3 | $1,3 \times 10^{-7}$ |
| D | 5 | $2,2 \times 10^{-7}$ |
| F | 8 | $7 \times 10^{-8}$ |
| clonidine | 6 | $3,2 \times 10^{-9}$ |

(*)n = nombre d'essais.

Il ressort de ce tableau que les composés de l'invention s'opposent à la tachycardie induite par stimulation électrique, à des concentrations très faibles.
Par ailleurs, en présence d'une concentration de $10^{-6}$ mole/l d'alpha-yohimbine la concentration du composé A nécessaire pour obtenir une réduction de 30% de la tachycardie est supérieure à $10^{-7}$mole/l. Ces résultats prouvent bien que les composés de l'invention agissent par un mécanisme spécifique via une action $\alpha_2$-agoniste.

c) Stimulation de l'iléon de cobaye.

Des fragments de muscles longitudinaux attachés à un indicateur de force isométrique, sont plongés dans une solution de Tyrode et sont tendus avec une force de 1 g (G.M. DREW, Brit.J.Pharmacol.64,(1978), 293-300; M. ANDREJAK et coll. Naunyn-Schmiedeberg's Arch.Pharmacol. 314,(1980),83-87).
La stimulation électrique des nerfs parasympatiques afférents aux fragments d'iléon provoque une contraction du muscle. Cette contraction est réduite en présence d'un $\alpha_2$-agoniste présynaptique et la réduction dépend de la concentration de l'agoniste utilisée. Cet effet est antagonisé par la présence simultanée d'un composé $\alpha_2$-antagoniste tel que l'alpha-yohimbine. Les composés à étudier ont été testés à des concentrations croissantes comprises entre $10^{-10}$ et $10^{-3}$ mole/l.
On détermine la concentration ($IC_{50}$ en mole/l) qui réduit de 50% l'intensité de la contraction du muscle.
Dans le tableau VII, on donne les concentrations $IC_{50}$ (en mole/l) obtenues pour les composés de l'invention. Ces résultats montrent que ces composés sont bien actifs à très faible concentration.

Tableau VII.

Inhibition de la contraction de l'iléon de cobaye.

| produits | n(*) | $IC_{50}$ (en mole/l) |
|---|---|---|
| A | 5 | $7 \times 10^{-9}$ |
| B | 6 | $3 \times 10^{-9}$ |
| E | 3 | $6 \times 10^{-5}$ |
| F | 4 | $7 \times 10^{-5}$ |
| I | 7 | $3 \times 10^{-7}$ |
| J | 4 | $3 \times 10^{-5}$ |
| L | 6 | $3 \times 10^{-8}$ |
| M | 5 | $4 \times 10^{-7}$ |
| P | 6 | $2 \times 10^{-9}$ |
| clonidine | 4 | $2 \times 10^{-8}$ |

(*)n = nombre d'essais.

En présence d'alpha-yohimbine, à la concentration de $10^{-6}$ mole /l, la concentration des composés A ou B par exemple, qui est nécessaire pour réduire de 50% l'intensité de la contraction du muscle, est plus élevée et devient supérieure à $10^{-6}$ mole/l, ce qui apporte une confirmation supplémentaire que les composés de l'invention agissent bien au niveau des récepteurs $\alpha_2$-adrénergiques pré-synaptiques.

4. Activité diurétique.

L'activité diurétique des composés de l'invention a été déterminée chez le chien Beagle (6 mâles et 6 femelles) au moyen d'une étude par permutations croisées randomisées à 6 voies.
Le composé à étudier est administré par voie intraveineuse à des doses croissantes: 2, 6,5, 20, 65 et 200 µg/kg.
Pendant les trois premières heures qui suivent l'injection on mesure le volume des urines excrétées.
Le tableau VIII donne pour le composé A, l'augmentation relative moyenne en % du volume d'urine excrétée par rapport au groupe des animaux qui n'ont pas reçu le produit.
Les résultats montrent que la dose minimale active qui provoque une augmentation statistiquement significative ($P < 0,05$) de l'excrétion urinaire est $\leqq$6,5 µg/kg chez les mâles, tandis que chez les femelles, cette dose est $\leqq$2 µg/kg.

Tableau VIII

Augmentation relative moyenne de l'excrétion urinaire (en %)

| dose (µg/kg) | mâles (n=6) | femelles (n=6) |
|---|---|---|
| 2 | 25 | 74 * |
| 6,5 | 122 * | 291 ** |
| 20 | 383 ** | 531 ** |
| 65 | 345 ** | 804 ** |
| 200 | 412 ** | 988 ** |

Analyse de la variance: * $P < 0,05$

** $P < 0,01$

n = nombre d'animaux

5. Toxicité.

La toxicité des composés de l'invention a été déterminée chez la souris mâle NMRI au moyen du test d'Irwin (S. IRWIN, Psychopharmacologia,13,(1968),222-257).
Des doses progressives du produit étudié sont administrées par voie intrapéritonéale à des groupes de trois souris jusqu'à ce que la dose mortelle soit atteinte (dose provoquant la mort de deux animaux sur trois dans les 48 heures).
Dans le tableau IX ci-dessous, on donne la dose mortelle observée pour les composés de l'invention. Il ressort de ce tableau que les composés de l'invention sont très peu toxiques.

Tableau IX.

Toxicité.

| Produits | Dose mortelle (en mg/kg) |
|---|---|
| A | 760 |
| B | 267 |
| C | 267 |
| D | 232 |
| E | 893 |
| F | 802 |
| G | 651 |
| H | 245 |
| I | > 270 |
| K | > 268 |
| L | 231 |
| M | > 288 |
| N | 284 |
| O | > 268 |
| P | 294 |

Les compositions pharmaceutiques renfermant les produits de l'invention peuvent être administrées par voie orale, parentérale ou rectale.

Les compositions pharmaceutiques utilisables pour l'administration orale peuvent être solides ou liquides, par exemple sous forme de comprimés (enrobés ou non), pilules, dragées, capsules en gélatine, solutions, sirops, etc. De même, les compositions utilisables pour l'administration par voie parentérale, sont les formes pharmaceutiquement connues pour ce genre d'administration, par exemple des solutions, des suspensions ou émulsions aqueuses ou huileuses. Pour l'administration par voie rectale, les compositions contenant les produits de l'invention se présentent généralement sous forme de suppositoires.

Les formes pharmaceutiques telles que solutions injectables, suspensions injectables, comprimés, gouttes, suppositoires, etc. sont préparées selon les méthodes couramment utilisées par les pharmaciens.

On mélange les composés de l'invention avec un véhicule solide ou liquide, non toxique, pharmaceutiquement acceptable, et éventuellement avec un agent dispersant, un agent désintégrant, un agent stabilisant, etc. On peut y ajouter, le cas échéant, des agents édulcorants, des agents colorants, etc.

Le pourcentage de produit actif dans les compositions pharmaceutiques peut varier dans des limites très larges, selon le patient et le mode d'administration, en particulier selon la fréquence d'administration. Quant à la posologie, elle peut varier dans une gamme très étendue de doses unitaires, par exemple de 3 à 350 μg de produit actif une ou deux fois par jour par voie intraveineuse, ou encore de 50 μg à 5 mg de produit actif une ou deux fois par jour par voie orale.

A titre d'exemple non limitatif d'une composition contenant un composé de l'invention, on donne ci-après

a) un exemple d'une solution stérile pour injection par voie intraveineuse:

| | |
|---|---|
| composé actif | 250 μg |
| acétate de sodium | 19,15 mg |
| acide acétique | 3,59 mg |
| chlorure de sodium | 81 mg |

eau pour injection ad 10 ml

(à conserver en ampoule brune de 10 ml, après filtration stérile de la solution)

b) un exemple de formule pour comprimé:

| | |
|---|---|
| composé actif | 0,5 mg |
| amidon de maïs | 38 mg |
| lactose | 63 mg |
| stéarate de magnésium | 1,2 mg |
| polyvinylpyrrolidone | 2,5 mg |

Ci-après, on donne des exemples, non limitatifs, de la préparation des 1-(1H-imidazol-4-yl)alkyl-benzamides substitués conformes à l'invention ainsi que de leurs composés intermédiaires. Dans ces exemples, les spectres de résonance magnétique nucléaire (RMN) ont été relevés avec un appareil Bruker à 250 MHz en utilisant le tétraméthylsilane comme étalon interne. Les déplacements chimiques sont indiqués en delta (ppm). Les lettres s, d, dd, t, q et m indiquent respectivement un singulet, un doublet, un double doublet, un triplet, un quadruplet et un multiplet.

Exemple 1. Préparation des 1-(1H-imidazol-4-yl)alkyl-benzoates d'alkyle de départ de formule II.

A. par estérification des acides correspondants (procédé (a)).

1. chlorhydrate de 2-hydroxy-5-[(1H-imidazol-4-yl)méthyl]-benzoate d'éthyle.

On sature à 0°C, par un courant d'acide chlorhydrique gazeux, une suspension de 3,1 g (12,2 mmoles) de chlorhydrate de l'acide 2-hydroxy-5-[(1H-imidazol-4-yl)méthyl]-benzoïque (préparé comme décrit ci-après à l'exemple 2.C.) dans 150 ml d'éthanol absolu. On chauffe ensuite lentement jusqu'à la température du reflux que l'on maintient pendant 10 heures. Ensuite, on évapore le solvant jusqu'à précipitation de l'ester. Celui-ci est filtré, lavé avec de l'éther diéthylique, puis séché. On obtient 2,3 g de chlorhydrate de 2-hydroxy-5-[(1H-imidazol-4-yl)méthyl]-benzoate d'éthyle.

Rendement: 68%. P.F.: 195-198°C.

Spectre RMN (DMSO): delta 1,34(3H,t), 4,03(2H,s), 4,37(2H,q), 6,95(1H,d), 7,40(1H,s), 7,49(1H,dd), 8,70(1H,d), 9,12(1H,s), 10,6(1H,s).

2. 2-hydroxy-3-[1-(1H-imidazol-4-yl)éthyl]-benzoate d'éthyle.

Ce composé a été préparé comme indiqué au point 1. ci-dessus, mais à partir de l'acide 2-hydroxy-3-[1-(1H-imidazol-4-yl)éthyl]-benzoïque (préparé comme décrit ci-après à l'exemple 2.A.2.). En fin de réaction, on neutralise le milieu réactionnel par addition d'une solution concentrée d'ammoniaque, on filtre les sels minéraux et on évapore le filtrat sous pression réduite. On purifie le résidu obtenu par chromatographie sur gel de silice (éluant: mélange dichlorométhane-méthanol 8:2 v/v). On obtient le 2-hydroxy-3-[1-(1H-imidazol-4-yl)éthyl]-benzoate d'éthyle avec un rendement de 35%. Le chlorhydrate correspondant fond à 168°C (éthanol-éther).
Analyse pour $C_{14}H_{16}N_2O_3$.HCl en %:

| | | | |
|---|---|---|---|
| calculé: | C 56,66 | H 5,40 | N 9,44 |
| trouvé: | 56,58 | 5,50 | 9,21 |

3. 2-hydroxy-3-[1-(1H-imidazol-4-yl)pentyl]-benzoate d'éthyle.

On chauffe à reflux pendant 9 heures, 1,18 g (3,8 mmoles) de chlorhydrate de l'acide 2-hydroxy-3-[1-(1H-imidazol-4-yl)pentyl]-benzoïque(préparé comme décrit ci-après à l'exemple 2.A.3.) en solution dans 15 ml d'orthoformiate de triéthyle, en présence de 1,2 g de montmorillonite K10 anhydre. Ensuite, on filtre et on évapore sous pression réduite. Le résidu obtenu est purifié par chromatographie sur 150 g de silice (éluant: mélange dichlorométhane-méthanol-ammoniaque 95:5:0,5 v/v/v).
On obtient 0,213 g de 2-hydroxy-3-[1-(1H-imidazol-4-yl)pentyl]-benzoate d'éthyle.
Spectre RMN(DMSO): delta 0,81(3H,t), 1,0-1,29(2H,m), 1,34(3H,t), 1,77-2,0(2H,m), 4,31-4,41(3H,t+q), 6,77(1H,s), 6,86(1H,t), 7,46-7,50(2H, d+s), 7,63(1H,d).
Le produit ainsi obtenu a été utilisé tel quel, sans autre purification, pour préparer le benzamide correspondant (exemple 4.15).

4. 2-hydroxy-3-[(1H-imidazol-4-yl)méthyl]-benzoate de méthyle.

Ce composé a été préparé comme indiqué au point 2. ci-dessus, mais à partir du chlorhydrate de l'acide 2-hydroxy-3-[(1H-imidazol-4-yl)méthyl]-benzoïque (préparé comme décrit ci-après à l'exemple 2.A.1.) et de méthanol.
P.F.: 153-154°C.
Spectre RMN(DMSO): delta 3,87(2H,m), 3,91(3H,s), 6,77(1H,s), 6,87(1H,t), 7,45(1H,dd), 7,58(1H,s), 7,69(1H,dd), 10(1H,s).

B. via la transformation de Claisen (procédés (b) et (c)).

1. 3-[(1H-imidazol-4-yl)méthyl]-2-méthoxybenzoate de méthyle.

1.a. 2-(2-chloro-2-propényloxy)-benzoate de méthyle.

On chauffe à reflux pendant 10 heures une suspension de 304 g (2 moles) de 2-hydroxybenzoate de méthyle, 25 g d'iodure de potassium, 69 g (0,5 mole) de carbonate de potassium et 69 g (0,625 mole) de 2,3-dichloropropène dans 3 litres d'acétone sèche. Après 2½ heures, 5 heures et 7½ heures de réaction, on rajoute chaque fois 69 g de carbonate de potassium et 69 g de 2,3-dichloropropène. Ensuite, on filtre la suspension et on évapore le filtrat sous pression réduite. Le résidu est repris par de l'acétate d'éthyle et la solution est lavée successivement avec une solution aqueuse saturée de thiosulfate de sodium, avec de l'eau et finalement avec une solution aqueuse saturée de chlorure de sodium. On sèche la phase organique sur du sulfate de sodium et on la distille sous pression réduite. On obtient ainsi 394 g de 2-(2-chloro-2-propényloxy)-benzoate de méthyle.
Rendement: 87%. P.Eb.: 119°C/1,3 mbar.

1.b.3-(2-chloro-2-propényl)-2-hydroxybenzoate de méthyle.

On dégaze soigneusement à l'argon, 274,1 g (1,21 mole) de 2-(2-chloro-2-propényloxy)-benzoate de méthyle placés dans un ballon de 2 litres. On chauffe ensuite le plus rapidement possible à 260°C. A cette température, une réaction exothermique se déclenche brutalement: la température monte d'elle-même jusqu'à 293°C et il y a reflux et noircissement du milieu réactionnel. Après refroidissement à la température ambiante, on distille le produit sous pression réduite. On obtient 241,1 g de 3-(2-chloro-2-propényl)-2-hydroxybenzoate de méthyle.
Rendement: 88%. P.Eb.: 109-110°C/1,3 mbar.

Spectre RMN ($CDCl_3$): delta 3,71(2H,s), 3,95(3H,s), 5,17(1H,m), 5,28(1H,m), 6,90(1H,t), 7,48(1H,dd), 7,85(1H,dd), 11,22(1H,s).

1.c. 3-(2-chloro-2-propényl)-2-méthoxybenzoate de méthyle.

Sans dépasser la température de 10°C, on ajoute par portions 8,81 g (306 mmoles) d'hydrure de sodium à une solution de 57,7 g (255 mmoles) de 3-(2-chloro-2-propényl)-2-hydroxybenzoate de méthyle dans 500 ml de diméthylformamide anhydre. On chauffe le mélange à 40°C pendant 15 minutes. On ajoute alors 43,45 g (306 mmoles) d'iodure de méthyle en solution dans 50 ml de toluène, puis on maintient la température du mélange à 40°C pendant 3 heures. On verse prudemment le mélange réactionnel dans 5 litres d'eau et on extrait plusieurs fois par l'acétate d'éthyle. On concentre la phase organique jusqu'à un volume de 500 ml, puis on la lave successivement avec de l'eau et avec une solution aqueuse saturée de chlorure de sodium. On sèche la solution sur du sulfate de sodium et on évapore le solvant sous pression réduite. Le résidu est purifié par chromatographie sur gel de silice (éluant: dichlorométhane-hexane 50/50 v/v). On obtient ainsi 49,2 g de 3-(2-chloro-2-propényl)-2-méthoxybenzoate de méthyle.
Rendement: 60%. P.Eb.: 107-110°C/0,5 mbar (huile).
Spectre RMN ($CDCl_3$): delta 3,73(2H,s), 3,82(3H,s), 3,92(3H,s), 5,14(1H,m), 5,32(1H,m), 7,15(1H,t), 7,47(1H,dd), 7,81(1H,dd).

1.d. 3-[(1H-imidazol-4-yl)méthyl]-2-méthoxybenzoate de méthyle.

On chauffe à reflux pendant 150 minutes 45,5 g (189 mmoles) de 3-(2-chloro-2-propényl)-2-méthoxybenzoate de méthyle et 81,5 g (378 mmoles) d'acide m-chloroperbenzoïque en solution dans 300 ml de chloroforme sec. On refroidit à 0°C et on élimine le précipité formé par filtration. On lave le filtrat successivement avec une solution aqueuse saturée de thiosulfate de sodium et avec une solution aqueuse saturée de bicarbonate de sodium. On sèche la solution sur du sulfate de sodium et on évapore le solvant sous pression réduite sans dépasser 30°C.
Le résidu obtenu est mis en suspension dans 300 ml de méthanol anhydre et mélangé à 111,3 g (1,32 mole) de bicarbonate de sodium finement broyé. On chauffe la mélange à reflux. On y ajoute par portions, d'heure en heure, 137,6 g (1,32 mole) d'acétate de formamidine. Après 5 heures et demie de chauffage à reflux, on élimine le méthanol sous pression réduite. On reprend le résidu par 500 ml d'eau et on extrait par de l'acétate d'éthyle. On sèche la phase organique sur du sulfate de sodium et on l'évapore sous vide. Le résidu est purifié par chromatographie sur gel de silice (éluant: mélange dichlorométhane-éthanol-ammoniaque 92,3:7:0,7 v/v/v). On obtient 13,8 g de 3-[(1H-imidazol-4-yl)méthyl]-2-méthoxybenzoate de méthyle.
Rendement: 30%.
Spectre RMN (DMSO): delta 3,73(3H,s), 3,83(3H,s), 3,89(2H,s), 6,78(1H,s), 7,11(1H,t), 7,24 à 7,73(3H,m).
Le produit ainsi obtenu a été utilisé tel quel, sans autre purification, pour préparer l'acide correspondant (exemple 2.B.1.).

Les composés suivants ont été préparés par la méthode décrite au point B.1. ci-dessus.

2. 2-hydroxy-3-[(1H-imidazol-4-yl)méthyl]-benzoate de méthyle.

Ce composé a été préparé à partir de 3-(2-chloro-2-propényl)-2-hydroxybenzoate de méthyle avec un rendement de 35,4%, en omettant bien entendu la méthylation à l'iodure de méthyle décrite au point 1.c. P.F.: 153-154°C.
Le produit obtenu est identique à celui préparé à l'exemple 1.A.4.

3. 3-[(1H-imidazol-4-yl)méthyl]-2-n-propoxybenzoate de méthyle.

Rendement: 20% (huile).
Le produit, obtenu à l'état brut, a été utilisé tel quel pour préparer l'acide correspondant (exemple 2.B.2.).

C. par réaction de Friedel et Crafts (procédé (d)).

1. chlorhydrate de 5-tert-butyl-2-hydroxy-3-[(1H-imidazol-4-yl)méthyl]-benzoate de méthyle.

On fait réagir 50 g de chlorhydrate de 1H-imidazole-4-méthanol avec 60 g de 5-tert-butyl-2-hydroxybenzoate de méthyle dans 150 ml d'acide sulfurique concentré à 20°C pendant 21 heures. On décompose prudemment

le mélange réactionnel sur de la glace.
Le produit solide obtenu est filtré et purifié par chromatographie, puis transformé en son chlorhydrate.
On obtient ainsi 1,1 g de chlorhydrate de 5-tert-butyl-2-hydroxy-3-[(1H-imidazol-4-yl)méthyl]-benzoate de méthyle.
Rendement: 2,8%. P.F.: 185-186°C.
Spectre RMN (DMSO): delta 1,3(9H,s), 3,45(3H,s), 4,1(2H,s), 7,3(1H,s), 7,75(2H,m), 9,0(1H,s), 10,5-13,0 (3H).

2. 2,6-dihydroxy-3-[(1H-imidazol-4-yl)méthyl]-benzoate de méthyle.

On mélange 95,84 g (0,57 mole) de 2,6-dihydroxybenzoate de méthyle, 190 ml d'acide formique et 51,14 g (0,38 mole) de chlorhydrate de 1H-imidazole-4-méthanol. On chauffe le mélange à reflux et pendant 15 minutes on distille l'azéotrope acide formique-eau. Ensuite, on maintient le chauffage à reflux pendant 17 heures. Le mélange réactionnel est versé dans de l'eau. On extrait le 2,6-dihydroxybenzoate de méthyle en excès par du toluène, puis on neutralise la phase aqueuse à pH 7-8 par addition d'une solution aqueuse saturée d'hydroxyde de sodium. On extrait par du dichlorométhane, on sèche les phases organiques sur du sulfate de sodium et on évapore le solvant sous pression réduite. Le résidu est purifié par chromatographie sur gel de silice (éluant: mélange dichlorométhane-méthanol-ammoniaque 94:6:0,6 v/v/v). On obtient 14,8 g de 2,6-dihydroxy-3-[(1H-imidazol-4-yl)méthyl]-benzoate de méthyle souillé par des traces de solvants résiduelles. Rendement 13%.
Spectre RMN (DMSO): delta 3,72(2H,s), 3,81(3H,s), 6,33(1H,d), 6,86(1H,s), 7,06(1H,d), 7,73(1H,s), 9,5 à 10,2(3H).
En raison de son instabilité, le produit ainsi obtenu a été utilisé tel quel, sans autre purification, pour préparer le benzamide correspondant (exemple 4.9.).
Les composés suivants ont été préparés par la méthode décrite au point C.2. ci-dessus.

3. 2,6-dihydroxy-3-[1-(1H-imidazol-4-yl)éthyl]-benzoate de méthyle.

Ce composé a été préparé à partir du chlorhydrate d'α-méthyl-1H-imidazole-4-méthanol. On maintient le chauffage à reflux pendant 19 heures. Le résidu finalement obtenu est purifié par chromatographie sur gel de silice (éluant: mélange dichlorométhane-méthanol-ammoniaque 95:5:0,5 v/v/v). Rendement: 43%.
Spectre RMN ($CDCl_3$): delta 1,52(3H,d), 4,0(3H,s), 4,48(1H,q), 6,40(1H,d), 6,79(1H,s), 7,14(1H,d), 7,47(1H,s), 10,0(3H).
Le produit obtenu a été utilisé tel quel, sans autre purification, pour préparer le benzamide correspondant (exemple 4.10).

4. 6-hydroxy-3-[(1H-imidazol-4-yl)méthyl]-2-méthylbenzoate d'éthyle et 2-hydroxy-3-[(1H-imidazol-4-yl)méthyl]-6-méthylbenzoate d'éthyle.

Ces deux composés ont été préparés simultanément à partir d'un mélange de 80,8 g (0,448 mole) de 2-hydroxy-6-méthylbenzoate d'éthyle, 225 ml d'acide formique et 51,5 g (0,382 mole) de chlorhydrate de 1H-imidazole-4-méthanol, maintenu à reflux pendant 53 heures. On sépare et on purifie les produits obtenus par chromatographie sur gel de silice (éluant: mélange dichlorométhane-méthanol-ammoniaque 94:6:0,5 v/v/v).
On obtient ainsi 7,2 g de 6-hydroxy-3-[(1H-imidazol-4-yl)méthyl]-2-méthylbenzoate d'éthyle. Rendement: 7,2%. P.F.: 42-45°C.
Spectre RMN ($CDCl_3$): delta 1,36(3H,t), 2,34(3H,s), 3,83(2H,s), 4,37(2H,q), 6,52(1H,s), 6,70(1H,d), 7,08(1H,d), 7,45(1H,s), 10,0(2H).
On obtient en même temps 2,8 g de 2-hydroxy-3-[(1H-imidazol-4-yl)méthyl]-6-méthylbenzoate d'éthyle. Rendement: 2,8%. P.F.: 101-103°C.
Spectre RMN ($CDCl_3$): delta 1,40(3H,t), 2,47(3H,s), 3,88(2H,s), 4,39(2H,q), 6,62(1H,d), 6,76(1H,s), 7,12(1H,d), 7,43(1H,s), 10,0(2H).

5. bromhydrate de 2-hydroxy-3-[(1H-imidazol-4-yl)méthyl]-4-méthylbenzoate de méthyle.

5.a. 5-bromo-2-hydroxy-3-[(1H-imidazol-4-yl)méthyl]-4-méthylbenzoate de méthyle.

A la température ambiante, on ajoute par portions 95,6 g (0,71 mole) de chlorhydrate de 1H-imidazole-4-méthanol à une solution de 87 g (0,355 mole) de 5-bromo-2-hydroxy-4-méthylbenzoate de méthyle (T.M. CRESP et al., J.Chem.Soc.Perkin,I,(1973),340) dans 900 ml d'acide sulfurique concentré. On maintient l'agitation pendant 234 heures. On verse ensuite le milieu réactionnel prudemment sur de la glace et on ramène le pH à 8 par addition d'une solution aqueuse saturée d'hydroxyde de sodium. On extrait par de l'acétate d'éthyle. La phase organique est évaporée sous pression réduite et le résidu est purifié par chromatographie sur 1,4 kg de silice (éluant: dichlorométhane-méthanol 9:1 v/v). Le produit ainsi obtenu est chromatographié une seconde fois sur 400 g de silice (éluant: mélange dichlorométhane-méthanol-ammoniaque 95:5:0,5 v/v/v). On obtient 2,4 g de 5-bromo-2-hydroxy-3-[(1H-imidazol-4-yl)méthyl]-4-méthylbenzoate de méthyle suffisamment pur pour être utilisé tel quel dans l'étape suivante.
Spectre RMN (DMSO): delta 2,43(3H,s), 3,90(3H,s), 3,96(2H,s), 6,62(1H,s), 7,51(1H,s), 7,87(1H,s).

5.b. bromhydrate de 2-hydroxy-3-[(1H-imidazol-4-yl)méthyl]-4-méthylbenzoate de méthyle.

On hydrogénolyse 2,38 g (7,2 mmoles) de 5-bromo-2-hydroxy-3-[(1H-imidazol-4-yl)méthyl]-4-méthylbenzoate de méthyle dissous dans 70 ml de méthanol, sous une pression d'hydrogène de 4,2 bars, à la température ambiante et en présence de 0,7 g de charbon palladié à 10%. On filtre le catalyseur et on évapore le solvant sous pression réduite. On obtient 1,83 g de bromhydrate de 2-hydroxy-3-[(1H-imidazol-4-yl)méthyl]-4-méthylbenzoate de méthyle. Rendement: 76%.
Spectre RMN (DMSO): delta 2,34(3H,s), 3,91(3H,s), 4,02(2H,s), 6,87(1H,s), 7,13(1H,s), 7,68(1H,d), 8,86(1H,s).
Le produit ainsi obtenu a été utilisé tel quel, sans autre purification, pour préparer le benzamide correspondant (exemple 4.14).

Exemple 2. Préparation des acides 1-(1H-imidazol-4-yl)alkyl-benzoïques de départ de formule IV.

A. par oxydation des 1-(1H-imidazol-4-yl)alkyl-benzèneméthanols correspondants.

1. Acide 2-hydroxy-3-[(1H-imidazol-4-yl)méthyl]-benzoïque (chlorhydrate).

On chauffe à 180°C, pendant deux heures et demie, sous bonne agitation, 1 g de 2-hydroxy-3-[(1H-imidazol-4-yl)méthyl]-benzèneméthanol (préparé selon la méthode décrite à l'exemple 6.1 de la demande de brevet européen n° 269.599 au nom de la demanderesse) en présence de 7,5 g d'hydroxyde de potassium. Ensuite, on refroidit le milieu réactionnel et on le dissout dans 10 ml d'eau. On acidifie la solution aqueuse à pH 3 à 4 par addition d'acide chlorhydrique concentré. On filtre le précipité qui s'est formé, on le sèche et on l'extrait par de l'alcool isopropylique à l'ébullition. L'alcool isopropylique est ensuite éliminé sous pression réduite et le résidu cristallin obtenu est recristallisé dans 5 ml d'une solution aqueuse d'acide chlorhydrique 1N. On obtient 0,74 g du chlorhydrate de l'acide 2-hydroxy-3-[(1H-imidazol-4-yl)méthyl]-benzoïque.
Rendement: 58%. P.F.: 257°C (décomp.).
Spectre RMN (DMSO): delta 4,08(2H,s), 6,09(1H,t), 7,37(1H,d), 7,52(1H,dd), 7,79(1H,dd), 9,05(1H,d).
Analyse pour $C_{11}H_{10}N_2O_3$.HCl en %

| | | | |
|---|---|---|---|
| calculé: | C 51,87 | H 4,32 | N 11,0 |
| trouvé: | 51,68 | 4,03 | 10,61 |

Ce composé est utilisé comme produit de départ à l'exemple 1.A.4.
On a préparé de la même manière le composé suivant:

2. Acide 2-hydroxy-3-[1-(1H-imidazol-4-yl)éthyl]-benzoïque.

Ce composé, utilisé comme produit de départ dans l'exemple 1.A.2., a été préparé au départ de 2-hydroxy-3-[1-(1H-imidazol-4-yl)éthyl] benzèneméthanol avec un rendement de 50%.
L'acide libre est obtenu par neutralisation du chlorhydrate et recristallisation dans l'eau. P.F.: 278-280°C.
Analyse pour $C_{12}H_{12}N_2O_3$ en %:

| | | | |
|---|---|---|---|
| calculé: | C 62,06 | H 5,17 | N 12,07 |
| trouvé: | 62,05 | 5,37 | 11,72 |

Le 2-hydroxy-3-[1-(1H-imidazol-4-yl)éthyl]-benzèneméthanol utilisé comme produit de départ a été préparé

selon la méthode décrite à l'exemple 6.5. de la demande de brevet européen n° 269.599 au nom de la demanderesse.

3. Acide 2-hydroxy-3-[1-(1H-imidazol-4-yl)pentyl]-benzoïque (chlorhydrate).

On chauffe à 170°C pendant 5 heures 3,86 g (14,8 mmoles) de 2-hydroxy-3-[1-(1H-imidazol-4-yl)pentyl]-benzèneméthanol (préparé selon la méthode décrite à l'exemple 6.6. de la demande de brevet européen n° 269.599 au nom de la demanderesse) en présence de 22 g d'hydroxyde de potassium. Ensuite, on refroidit le milieu réactionnel et on le dissout dans 100 ml d'eau. On filtre l'insoluble et on acidifie le filtrat à pH 10 par addition d'acide chlorhydrique concentré. On élimine les sels qui se sont formés par filtration et on acidifie finalement le filtrat jusqu'à pH 1. Le chlorhydrate de l'acide 2-hydroxy-3-[1-(1H-imidazol-4-yl)pentyl]benzoïque précipite. On le recristallise dans 100 ml d'alcool isopropylique. On obtient 1,44 g de produit. P.F.: 239-251°C.
Spectre RMN (DMSO): delta 0,83(3H,t), 1,17 à 1,32(4H,m), 1,94 à 2,07(2H,m), 4,43(1H,t), 6,79(1H,t), 7,33(1H,d), 7,47(1H,s), 7,69(1H,d), 8,84(1H,s).
Ce composé est utilisé comme produit de départ à l'exemple 1.A.3.

B. par hydrolyse des esters correspondants préparés via la transformation de Claisen.

1. Acide 3-[(1H-imidazol-4-yl)méthyl]-2-méthoxybenzoïque.

On chauffe à reflux pendant 3 heures 4,3 g (17,5 mmoles) de 3-[(1H-imidazol-4-yl)méthyl]-2-méthoxybenzoate de méthyle (préparé à l'exemple 1.B.1.) en solution dans 20 ml de méthanol et 21 ml d'une solution aqueuse d'hydroxyde de sodium 1N. On évapore le méthanol sous pression réduite et on acidifie la solution aqueuse jusqu'à pH 5 par addition de 21 ml d'une solution aqueuse d'acide chlorhydrique 1N. On filtre le précipité, on concentre le filtrat à la moitié de son volume et on filtre le nouveau précipité qui est apparu. Les deux jets sont lavés à l'hexane et séchés sous vide. On obtient 3,16 g de l'acide 3-[(1H-imidazol-4-yl)méthyl]-2-méthoxybenzoïque pratiquement pur. Rendement: 78%.
Spectre RMN (DMSO + $CF_3COOH$): delta 3,78(3H,s), 4,07(2H,s), 6,96 à 7,82(4H,m), 8,91(1H,s).
Le produit obtenu a été utilisé tel quel pour préparer le benzamide correspondant (exemple 5.1.).
On a préparé de la même manière le composé suivant:

2. Acide 3-[(1H-imidazol-4-yl)méthyl]-2-n-propoxybenzoïque.

Il est obtenu à partir du 3-[(1H-imidazol-4-yl)méthyl]-2-n-propoxybenzoate de méthyle (préparé à l'exemple 1.B.3.)
Le produit obtenu a été utilisé tel quel pour préparer le benzamide correspondant (exemple 5.2.).

C. par hydrolyse des esters correspondants préparés par réaction de Friedel et Crafts.

Acide 2-hydroxy-3-[(1H-imidazol-4-yl)méthyl]-benzoïque et acide 2-hydroxy-5-[(1H-imidazol-4-yl)méthyl]-benzoïque (chlorhydrate).
On ajoute par portions 181 g (1,35 mole) de chlorhydrate de 1H-imidazole-4-méthanol à un mélange de 156 ml (1,2 mole) de 2-hydroxybenzoate de méthyle et de 675 g d'acide polyphosphorique chauffé à 80°C.
On maintient le milieu réactionnel sous bonne agitation à cette température pendant 288 heures. Ensuite, on décompose le mélange sur de la glace et on extrait 2 fois par du toluène.
La phase aqueuse est alcalinisée jusqu'à pH 9,5 par addition de 790 ml d'une solution aqueuse saturée d'hydroxyde de sodium. Les sels minéraux qui précipitent sont éliminés par filtration et lavés par du méthanol. Cette solution méthanolique de lavage est réunie à la phase aqueuse et le mélange est concentré par élimination partielle du méthanol. Ensuite, on alcalinise la solution jusqu'à pH 10,3 par addition d'une solution aqueuse d'hydroxyde de sodium 10N et on chauffe à 100°C pendant une heure et demie pour saponifier les esters. On neutralise la solution aqueuse jusqu'à pH 7,5 par addition d'acide chlorhydrique 10N, on filtre sur norite et on évapore le filtrat sous pression réduite. Le résidu est repris trois fois de suite par un mélange toluène-éthanol et séché par distillation azéotropique. Il est ensuite partiellement dissous dans du méthanol chaud et débarrassé des sels minéraux insolubles par filtration. On évapore le filtrat sous pression réduite, on redissout le résidu dans un minimum d'eau et on purifie par passage sur une colonne d'Amberlite IR93 (hauteur de la colonne: 60 cm; diamètre: 8 cm; équivalence: 2,64 mole). Le 1H-imidazole-4-méthanol en excès ainsi que ses polymères sont élués par de l'eau (le pH de l'éluat varie de 11,2 à 7,3). Ensuite l'élution est poursuivie avec une solution aqueuse d'acide chlorhydrique à 4%.

On ramène l'éluat acide (9 litres) à pH 7,7 par addition d'une solution aqueuse saturée d'hydroxyde de sodium, puis on l'évapore sous pression réduite. On sèche à nouveau le résidu obtenu par distillation azéotropique avec un mélange toluène-éthanol et on le reprend par 1,6 litre d'acétonitrile. Puis, on filtre. Le résidu sur le filtre (129 g) est chromatographié sur silice (800 g, 15 μm) après avoir été, au préalable, déposé sur 300 g de silice (0,2 à 0,5 mm) (éluant: mélange acétate d'éthyle-éthanol 75:25 v/v).
On obtient ainsi 5,99 g de l'acide 2-hydroxy-3-[(1H-imidazol-4-yl)méthyl]-benzoïque. P.F.: 245-252°C (eau).
Analyse pour $C_{11}H_{10}N_2O_3$ en %

| | | | |
|---|---|---|---|
| calculé: | C 60,56 | H 4,59 | N 12,04 |
| trouvé: | 60,32 | 4,69 | 12,41 |

On obtient en même temps 31 g de l'acide 2-hydroxy-5-[(1H-imidazol-4-yl)méthyl]-benzoïque. Son chlorhydrate, utilisé comme produit de départ à l'exemple 1.A.1., fond à 254-258°C (méthanol-éther éthylique).
Analyse pour $C_{11}H_{10}N_2O_3$. HCl en %:

| | | | | |
|---|---|---|---|---|
| calculé: | C 51,87 | H 4,32 | N 11,0 | $Cl^-$ 13,40 |
| trouvé: | 51,65 | 4,24 | 10,45 | 13,73 |

Exemple 3. Préparation des 2-hydroxy-3-[1-(1H-imidazol-4-yl)alkyl]-benzonitriles de départ de formule V.

1. 2-hydroxy-3-[(1H-imidazol-4-yl)méthyl]-benzonitrile.

1.a. 4-benzyloxy-2-[(1H-imidazol-4-yl)méthyl]-3-oxo-butanoate d'éthyle.

A la température de 10°C, on ajoute en une fois 182 g (0,77 mole) de 4-benzyloxy-3-oxo-butanoate d'éthyle à une solution de 16,9 g (0,735 mole) de sodium dans 590 ml d'éthanol absolu.
On agite le mélange pendant 45 minutes à la température ambiante, puis on refroidit à -45°C et on y ajoute en une fois une solution de 53,6 g (0,35 mole) de chlorhydrate de 4-chlorométhyl-1H-imidazole dans 300 ml d'éthanol absolu. On laisse le mélange revenir à la température ambiante et on agite pendant une heure. Ensuite, on évapore la suspension à sec. On reprend le résidu par une solution de 35 ml d'acide chlorhydrique concentré dans 900 ml d'eau et on extrait plusieurs fois par l'éther diéthylique. On neutralise la phase aqueuse par une solution de 18 g d'hydroxyde de sodium dans 200 ml d'eau et on extrait plusieurs fois par de l'acétate d'éthyle. On lave les phases organiques à l'eau et avec une solution aqueuse saturée de chlorure de sodium. On sèche sur du sulfate de sodium et on évapore sous pression réduite. On obtient 107 g de 4-benzyloxy-2-[(1H-imidazol-4-yl)méthyl]-3-oxo-butanoate d'éthyle pratiquement pur. Rendement: 97%.
Spectre RMN (DMSO): delta 1,11(3H,t), 2,98(2H,m), 4,05(2H,q), 4,08(1H,m), 4,25(2H,dd), 4,47(2H,s), 6,75(1H,s), 7,25 à 7,39(5H,m), 7,47(1H,d).

1.b. 4-benzyloxy-3-hydroxy-2-[(1H-imidazol-4-yl)méthyl]-butanoate d'éthyle.

A une solution de 101,2 g (0,32 mole) de 4-benzyloxy-2-[(1H-imidazol-4-yl)méthyl]-3-oxo-butanoate d'éthyle dans 600 ml d'éthanol, refroidie à -20°C, on ajoute en une fois une solution glacée de 6,03 g (0,16 mole) de borohydrure de sodium dans 25 ml d'eau. On ramène le mélange à la température ambiante et on agite pendant une heure. On ajoute ensuite 25 ml d'acétone. On évapore la solution à sec et on reprend le résidu par 500 ml d'eau. On extrait plusieurs fois par de l'acétate d'éthyle. Les phases organiques sont lavées à l'eau et avec une solution aqueuse saturée de chlorure de sodium. On sèche sur du sulfate de sodium et on évapore le solvant sous pression réduite. Le résidu est purifié par chromatographie sur gel de silice (éluant: mélange dichlorométhane-méthanol-ammoniaque: 93,5:6:0,5 v/v/v). On obtient 95,8 g de 4-benzyloxy-3-hydroxy-2-[(1H-imidazol-4-yl)méthyl]-butanoate d'éthyle (mélange de diastéréoisomères).
Rendement: 94%.
Spectre RMN ($CDCl_3$): delta 1,15 et 1,16(3H,2t), 2,90 à 3,05(3H,m), 3,51 à 3,58(2H,m), 3,96 à 4,11(3H,2q + 1m), 4,51 et 4,53(2H,2s), 6,73 et 6,75(1H,2s), 7,25 à 7,36(5H,m).

1.c. chlorhydrate de 4-hydroxy-3-[(1H-imidazol-4-yl)méthyl]-dihydro-2(3H)-furanone.

On hydrogénolyse 93,9 g (0,295 mole) de 4-benzyloxy-3-hydroxy-2-[(1H-imidazol-4-yl)méthyl]-butanoate

d'éthyle en solution dans 500 ml d'éthanol absolu et 65 ml d'une solution d'acide chlorhydrique 6,8N dans l'éthanol, en présence de 5 g de charbon palladié à 10% et sous une pression d'hydrogène de 3,5 bars. Puis, on filtre le catalyseur et on élimine le solvant à 65°C sous pression réduite. On obtient 67,1 g de chlorhydrate de 4-hydroxy-3-[(1H-imidazol-4-yl)méthyl]-dihydro-2(3H)-furanone (mélange de diastéréoisomères). Rendement pratiquement quantitatif.
Le produit a été utilisé tel quel dans l'étape suivante.

1.d.3-[(1H-imidazol-4-yl)méthyl]-2(5H)-furanone.

On chauffe pendant 75 minutes à 160°C sous une pression de 0,0013 mbar, 67,1 g (0,295 mole) de chlorhydrate de 4-hydroxy-3-[(1H-imidazol-4-yl)méthyl]-dihydro-2(3H)-furanone. On refroidit et on reprend par 125 ml d'éthanol absolu. On neutralise par addition de 70 ml d'une solution d'ammoniac 5N dans l'éthanol. On filtre la suspension et on élimine le solvant sous pression réduite. Le résidu est purifié par chromatographie sur gel de silice (éluant: mélange dichlorométhane-méthanol-ammoniaque 91,5:8:0,5 v/v/v). Après recristallisation dans l'acétonitrile, on obtient 27,5 g de 3-[(1H-imidazol-4-yl)méthyl]-2(5H)-furanone. Rendement: 53% (calculé sur les étapes 1.c. et 1.d. réunies). P.F.: 123°C.
Spectre RMN ($CDCl_3$): delta 3,63(2H,q), 4,79(2H,q), 6,90(1H,d), 7,25(1H,quintuplet), 7,52(1H,d).

1.e. 2-hydroxy-3-[(1H-imidazol-4-yl)méthyl]-benzonitrile.

A une suspension de 24,6 g (0,15 mole) de 3-[(1H-imidazol-4-yl)méthyl]-2(5H)-furanone dans 225 ml d'acrylonitrile anhydre, on ajoute successivement 63 ml (0,45 mole) de triéthylamine anhydre et 57 ml (0,45 mole) de triméthylchlorosilane. On chauffe le mélange à reflux (72-74°C) pendant quatre heures. Ensuite, on évapore sous pression réduite. Le résidu est traité en une fois par 75 ml d'acide bromhydrique concentré et est maintenu à 80°C pendant deux minutes. La solution est ensuite versée sur de la glace, diluée par addition de 300 ml d'acétate d'éthyle et 300 ml d'eau, puis neutralisée par du bicarbonate de sodium solide. On filtre sur célite et le filtrat est extrait plusieurs fois par de l'acétate d'éthyle. Les phases organiques sont lavées à l'eau et par une solution aqueuse saturée de chlorure de sodium, puis séchées sur du sulfate de sodium et évaporées sous pression réduite. Le résidu obtenu est trituré dans de l'éther diéthylique. On obtient 22,8 g de 2-hydroxy-3-[(1H-imidazol-4-yl)méthyl]-benzonitrile. Rendement: 76%.
Spectre RMN (DMSO): delta 3,90(2H,s), 6,88(1H,t), 7,06(1H,s), 7,41(1H,dd), 7,47(1H,dd), 7,99(1H,d).
Son chlorhydrate fond à 245°C.

2. 2-hydroxy-3-[1-(1H-imidazol-4-yl)éthyl]-benzonitrile.

2.a. 4-benzyloxy-2-[1-(1H-imidazol-4-yl)éthyl]-3-oxo-butanoate d'éthyle.

Ce composé a été préparé comme indiqué en 1.a. ci-dessus à partir de 4-benzyloxy-3-oxo-butanoate d'éthyle et de 4-(1-chloroéthyl)-1H-imidazole.
Rendement: 68% (mélange de diastéréoisomères).
Spectre RMN ($CDCl_3$): delta 1,10 et 1,22(3H,2t), 1,33 et 1,36(3H,2d), 3,60 à 3,72(1H,m), 3,93 à 4,20(3H,m), 4,47 et 4,55(1H,2s), 6,70 et 6,74(1H,2s), 7,26 à 7,35(5H,m), 7,41 et 7,45(1H,s+d).

2.b. 4-benzyloxy-3-hydroxy-2-[1-(1H-imidazol-4-yl)éthyl]-butanoate d'éthyle.

Ce composé a été préparé comme indiqué en 1.b. ci-dessus par réduction du 4-benzyloxy-2-[1-(1H-imidazol-4-yl)éthyl]-3-oxo-butanoate d'éthyle. Rendement 91% (mélange de diastéréoisomères).
Spectre de masse: 332(M+), 314, 287, 211, 181, 135, 95, 91.

2.c. chlorhydrate de 4-hydroxy-3-[1-(1H-imidazol-4-yl)éthyl]-dihydro-2(3H)-furanone.

Ce composé a été préparé comme indiqué en 1.c. ci-dessus par hydrogénolyse du 4-benzyloxy-3-hydroxy-2-[1-(1H-imidazol-4-yl)éthyl]-butanoate d'éthyle. Rendement pratiquement quantitatif. On obtient un mélange de diastéréoisomères qui est utilisé tel quel dans l'étape suivante.

2.d. 3-[1-(1H-imidazol-4-yl)éthyl]-2(5H)-furanone.

On chauffe pendant une heure à 170°C, sous une pression de 13,3 mbars, 75,1 g (0,32 mole) de chlorhydrate

de 4-hydroxy-3-[1-(1H-imidazol-4-yl)éthyl]-dihydro-2(3H)-furanone dans 30 ml d'éthylène glycol. On élimine ensuite le solvant sous une pression de 0,0013 mbar. On reprend le résidu dans 300 ml d'éthanol absolu et on neutralise par addition de 63,2 ml d'une solution d'ammoniac 5N dans l'éthanol. On filtre la suspension et on évapore le solvant sous pression réduite. Le résidu est purifié par chromatographie sur gel de silice (éluant: mélange dichlorométhane-méthanol-ammoniaque 91,5:8:0,5 v/v/v). Après recristallisation dans l'acétonitrile, on obtient 41,9 g de 3-[1-(1H-imidazol-4-yl)éthyl]-2(5H)-furanone. Rendement: 74% (calculé sur les étapes 2.c. et 2.d. réunies). P.F.: 127-129°C.
Spectre RMN (DMSO): delta 1,40(3H,d), 3,72(1H,q), 4,84(2H,t), 6,80(1H,t), 7,35(1H,q), 7,51(1H,s).

2.e. 2-hydroxy-3-[1-(1H-imidazol-4-yl)éthyl]-benzonitrile.

A une suspension de 17,8 g (0,1 mole) de 3-[1-(1H-imidazol-4-yl)éthyl]-2(5H)-furanone dans 150 ml d'acrylonitrile anhydre, on ajoute successivement 56 ml (0,4 mole) de triéthylamine anhydre et 50,7 ml (0,4 mole) de triméthylchlorosilane. On chauffe le mélange à reflux pendant trois heures et demie. Ensuite, on évapore le mélange réactionnel sous pression réduite. Le résidu est traité en une fois par 50 ml d'acide chlorhydrique concentré et est maintenu à 80°C pendant deux minutes. La solution est ensuite versée sur de la glace, neutralisée par une solution aqueuse saturée de bicarbonate de sodium et extraite plusieurs fois par de l'acétate d'éthyle. Les phases organiques sont lavées à l'eau et avec une solution aqueuse saturée de chlorure de sodium, puis séchée sur du sulfate de sodium et évaporée sous pression réduite. Le résidu est purifié par chromatographie sur gel de silice (éluant: mélange dichlorométhane-méthanol-ammoniaque 93,5:6:0,5 v/v/v). On obtient 17,6 g de 2-hydroxy-3-[1-(1H-imidazol-4-yl)éthyl]-benzonitrile pratiquement pur.
Rendement: 83%. P.F.: 172°C.
Spectre RMN (DMSO): delta 1,52(3H,d), 4,21(1H,q), 6,86(1H,t), 7,02(1H,s), 7,41(1H,dd), 7,44(1H,dd), 7,85(1H,s).

Exemple 4. Préparation des 1-(1H-imidazol-4-yl)alkyl-benzamides de formule I par réaction des esters de formule II avec un composé azoté de formule III.

1. chlorhydrate de 2-hydroxy-3-[(1H-imidazol-4-yl)méthyl]-benzamide.

On fait passer un courant de gaz ammoniac, séché sur de l'hydroxyde de potassium, dans une solution de 18,1 g (78 mmoles) de 2-hydroxy-3-[(1H-imidazol-4-yl)méthyl]-benzoate de méthyle (préparé à l'exemple 1.B.2. et à l'exemple 1.A.4.) dans 400 ml de méthanol anhydre, pendant une nuit. Ensuite, on chauffe à reflux pendant 2 heures. On évapore le mélange réactionnel sous pression réduite et le résidu est purifié par chromatographie sur gel de silice (éluant: mélange dichlorométhane-méthanol-ammoniaque 89,5:10:0,5 v/v/v).
On obtient 16,6 g de 2-hydroxy-3-[(1H-imidazol-4-yl)méthyl]-benzamide. Rendement: 98%. P.F.: 197,6°C.
Analyse pour $C_{11}H_{11}N_3O_2$ en %:

| | | | |
|---|---|---|---|
| calculé: | C 60,83 | H 5,07 | N 19,35 |
| trouvé: | 60,91 | 5,06 | 19,32 |

L'amide, traité dans l'éthanol avec 1,2 équivalent d'acide chlorhydrique, forme un chlorhydrate avec un rendement de 73%.
P.F.: 287,8°C.
Analyse pour $C_{11}H_{11}N_3O_2.HCl$ en %:

| | | | | |
|---|---|---|---|---|
| calculé: | C 52,07 | H 4,73 | N 16,57 | $Cl^-$ 14,00 |
| trouvé: | 52,04 | 4,76 | 16,54 | 13,94 |

2. 2-hydroxy-5-[(1H-imidazol-4-yl)méthyl]-benzamide.

Ce composé a été préparé comme indiqué en 1. ci-dessus à partir du chlorhydrate de 2-hydroxy-5-[(1H-imidazol-4-yl)méthyl]-benzoate d'éthyle (préparé à l'exemple 1.A.1.). Le milieu réactionnel est agité pendant 3 jours à la température ambiante. Le produit de réaction est purifié par chromatographie sur gel de silice (éluant: dichlorométhane-méthanol 85:15 v/v)
P.F.: 180-185°C (alcool isopropylique).

Analyse pour $C_{11}H_{11}N_3O_2$ en %:

| | | | |
|---|---|---|---|
| calculé: | C 60,83 | H 5,07 | N 19,35 |
| trouvé: | 60,71 | 5,25 | 19,01 |

3. Chlorhydrate de 2-hydroxy-3-[(1H-imidazol-4-yl)méthyl]-N-méthylbenzamide.

On chauffe en autoclave à 75°C pendant 3 heures 6,96 g (30 mmoles) de 2-hydroxy-3-[(1H-imidazol-4-yl)méthyl]-benzoate de méthyle (préparé à l'exemple 1.B.2.) et 60 ml de méthylamine en solution dans 350 ml d'éthanol. On évapore le mélange sous pression réduite. On reprend le résidu par de l'eau et on extrait 3 fois par de l'acétate d'éthyle. On sèche la phase organique sur du sulfate de sodium et on évapore le solvant sous pression réduite. Le résidu est purifié par chromatographie sur gel de silice (éluant: mélange dichlorométhane-méthanol-ammoniaque 91,5:8,0:0,5 v/v/v).
Après évaporation des solvants, le produit obtenu cristallise dans l'acétate d'éthyle. On obtient 4,95 g de 2-hydroxy-3-[(1H-imidazol-4-yl)méthyl]-N-méthylbenzamide. Rendement: 71%.
L'amide, traité dans un mélange éthanol-éther avec 1,2 équivalent d'acide chlorhydrique, fournit 4,7 g de chlorhydrate.
Rendement: 59%. P.F.: 233,9°C.
Analyse pour $C_{12}H_{13}N_3O_2.HCl$ en %

| | | | |
|---|---|---|---|
| calculé: | C 53,83 | H 5,23 | N 15,70 |
| trouvé: | 53,74 | 5,17 | 15,55 |

4. 2-hydroxy-3-[(1H-imidazol-4-yl)méthyl]-benzohydrazide.

On chauffe à reflux pendant 13 heures un mélange de 10 g (43,1 mmoles) de 2-hydroxy-3-[(1H-imidazol-4-yl)méthyl]-benzoate de méthyle (préparé à l'exemple 1.B.2.) et de 4,31 g (86,2 mmoles) d'hydrate d'hydrazine en solution dans 100 ml de méthanol. On y rajoute encore 2,15 g (43,1 mmoles) d'hydrate d'hydrazine et on maintient le chauffage à reflux encore pendant 24 heures. Puis, on évapore le mélange sous pression réduite. On reprend le résidu par 100 ml d'eau (pH = 8). On sature la solution avec du chlorure de sodium et on extrait avec de l'acétate d'éthyle. On sèche la phase organique sur du sulfate de sodium et on évapore le solvant sous pression réduite. Le résidu cristallise dans l'éthanol. On obtient 6,1 g de 2-hydroxy-3-[(1H-imidazol-4-yl)méthyl]-benzohydrazide, recristallisable dans le méthanol.
Rendement: 61%. P.F.: 189,7°C.
Analyse pour $C_{11}H_{12}N_4O_2$ en %

| | | | |
|---|---|---|---|
| calculé: | C 56,88 | H 5,21 | N 24,13 |
| trouvé: | 56,91 | 5,24 | 24,00 |

5. chlorhydrate de 2-hydroxy-3-[1-(1H-imidazol-4-yl)éthyl]-benzamide.

On agite pendant 90 heures à la température ambiante et en présence d'une quantité catalytique de 20 mg de méthylate de sodium, une solution de 2 g de 2-hydroxy-3-[1-(1H-imidazol-4-yl)éthyl]-benzoate d'éthyle (préparé à l'exemple 1.A.2.) dans 50 ml de méthanol, dans laquelle on fait passer un courant gazeux d'ammoniac. Ensuite, on évapore le méthanol et le résidu est purifié par chromatographie sur gel de silice (éluant: dichlorométhane, méthanol, ammoniaque 8,5:1:0,5 v/v/v). Le produit obtenu est transformé en chlorhydrate dans une solution éthanolique d'acide chlorhydrique en présence d'éther diéthylique. On obtient 1,2 g de chlorhydrate de 2-hydroxy-3-[1-(1H-imidazol-4-yl)éthyl]-benzamide.
Rendement: 68%. P.F.: 240-243°C.
Analyse pour $C_{12}H_{13}N_3O_2.HCl$ en %:

| | | | | |
|---|---|---|---|---|
| calculé: | C 53,83 | H 4,86 | N 15,70 | $Cl^-$ 13,27 |
| trouvé: | 54,0 | 4,88 | 15,73 | 13,17 |

L'amide obtenu après purification par chromatographie sur gel de silice est résolu en ses deux énantiomères par chromatographie sur une phase chirale d'alpha-glycoprotéine (éluant: alcool isopropylique-tampon phosphate 0,02 M, pH 7; 1:99 v/v). On transforme ensuite chacun des énantiomères de l'amide en chlorhydrate correspondant de la manière indiquée ci-dessus. On obtient ainsi en quantités quasi égales:

a) le chlorhydrate de (+)-2-hydroxy-3-[1-(1H-imidazol-4-yl)éthyl]-benzamide hydraté: P.F.: 107,8°C (eau). $[\alpha]_D^{25}$ = + 82,04° (c = 1, méthanol).

Spectre RMN (DMSO): delta 1,57(3H,d), 3,30(5H,m), 4,56(1H,q), 6,83(1H,t), 7,23(1H,dd), 7,40(1H,s), 7,82(1H,dd), 7,92(1H,m), 8,51(1H,m), 8,98(1H,d), 13,5 à 14,5(2H,m).

b) le chlorhydrate de (-)-2-hydroxy-3-[1-(1H-imidazol-4-yl)éthyl]-benzamide hydraté: P.F.: 107,4°C (eau). $[\alpha]_D^{25}$ = -79,13° (c = 1, méthanol).

Spectre RMN: identique au précédent.

6. 2-hydroxy-5-[(1H-imidazol-4-yl)méthyl]-N-méthylbenzamide.

Ce composé a été préparé selon le procédé décrit au point 3. ci-dessus à partir du chlorhydrate de 2-hydroxy-5-[(1H-imidazol-4-yl)méthyl]-benzoate d'éthyle (préparé à l'exemple 1.A.1.) et de méthylamine. Au préalable, on libère la base à partir du chlorhydrate par un léger excès (1,2 équivalent) de méthylate de sodium. Le produit obtenu après évaporation du solvant, est purifié par chromatographie sur gel de silice (éluant: mélange dichlorométhane-éthanol-ammoniaque 97,5:12:0,5 v/v/v).
Rendement: 67% (après recristallisation dans l'éthanol). P.F.: 219,5°C.
Analyse pour $C_{12}H_{13}N_3O_2$ en %:

| | | | |
|---|---|---|---|
| calculé: | C 62,32 | H 5,67 | N 18,17 |
| trouvé: | 62,23 | 5,65 | 18,06 |

Spectre RMN (DMSO): delta 2,80(3H,d), 3,27(1H,s), 3,76(2H,s), 6,67(1H,s), 6,80(1H,d), 7,23(1H,dd), 7,49(1H,s), 7,87(1H,d), 8,80(1H,s), 11,8(1H,s).

7. 1-[2-hydroxy-5-[(1H-imidazol-4-yl)méthyl]benzoyl]-pyrrolidine.

On chauffe à reflux pendant 30 minutes 300 mg de chlorhydrate de 2-hydroxy-5-[(1H-imidazol-4-yl)méthyl]-benzoate d'éthyle (préparé à l'exemple 1.A.1.) en solution dans 5 ml de pyrrolidine. On élimine l'excès d'amine sous pression réduite et on purifie le résidu par chromatographie sur silice (éluant: mélange dichlorométhane-éthanol-ammoniaque 88,5:11:0,5 v/v/v). On obtient 120 mg de 1-[2-hydroxy-5-[(1H-imidazol-4-yl)méthyl]benzoyl]-pyrrolidine. P.F.: 96-98°C. Rendement: 36%.
Spectre RMN (DMSO): delta 1,81(4H,s), 3,35(4H,s), 3,74(2H,s), 6,70(1H,s), 6,78(1H,d), 7,04(1H,d), 7,08(1H,dd), 7,50(1H,s), 9,85(1H,s), 11,8(1H,s).

8. 5-tert-butyl-2-hydroxy-3-[(1H-imidazol-4-yl)méthyl]-benzamide.

Ce composé a été préparé comme indique en 2. ci-dessus à partir du chlorhydrate de 5-tert-butyl-2-hydroxy-3-[(1H-imidazol-4-yl)méthyl]-benzoate de méthyle (préparé à l'exemple 1.C.1.).
Rendement: 22,7%. P.F.: 209-211°C (tétrahyrofuranne).
Analyse pour $C_{15}H_{19}N_3O_2$ en %

| | | | |
|---|---|---|---|
| calculé: | C 65,91 | H 7,00 | N 15,37 |
| trouvé: | 65,42 | 7,06 | 15,14 |

9. chlorhydrate de 2,6-dihydroxy-3-[(1H-imidazol-4-yl)méthyl]-benzamide.

Ce composé a été préparé comme indiqué en 1. ci-dessus à partir du 2,6-dihydroxy-3-[(1H-imidazol-4-yl)méthyl]-benzoate de méthyle (préparé à l'exemple 1.C.2.). On fait passer le courant de gaz ammoniac pendant trois heures dans la solution maintenue à température ambiante, puis on évapore. Le résidu est recristallisé dans le dioxanne. Rendement: 81%.

L'amide, traité dans l'éthanol avec 1,2 équivalent d'acide chlorhydrique, forme un chlorhydrate avec un rendement de 73%. P.F.: 290,3°C (décomp.).
Analyse pour $C_{11}H_{11}N_3O_3$.HCl en %:

| | | | | |
|---|---|---|---|---|
| calculé: | C 48,99 | H 4,49 | N 15,58 | $Cl^-$ 13,15 |
| trouvé: | 48,79 | 4,43 | 15,44 | 13,16 |

10. chlorhydrate de 2,6-dihydroxy-3-[1-(1H-imidazol-4-yl)éthyl]-benzamide.

Ce composé a été préparé comme indiqué en 9. ci-dessus à partir du 2,6-dihydroxy-3-[1-(1H-imidazol-4-yl) éthyl]-benzoate de méthyle (préparé à l'exemple 1.C.3.). Le résidu est recristallisé dans le toluène. Rendement: 85%.
L'amide, traité dans un mélange éthanol-éther avec 1,1 équivalent d'acide chlorhydrique, forme un chlorhydrate. Rendement: 77% (après recristallisation dans l'eau). P.F.: 288,8°C (décomp.).
Analyse pour $C_{12}H_{13}N_3O_3$.HCl en %:

| | | | | |
|---|---|---|---|---|
| calculé: | C 50,80 | H 4,97 | N 14,81 | $Cl^-$ 12,50 |
| trouvé: | 50,68 | 4,92 | 14,67 | 12,09 |

11. chlorhydrate de 6-hydroxy-3-[(1H-imidazol-4-yl)méthyl]-2-méthylbenzamide.

On chauffe en autoclave, à 60°C pendant 72 heures, une solution de 6,1 g (23,4 mmoles) de 6-hydroxy-3-[(1H-imidazol-4-yl)méthyl]-2-méthylbenzoate d'éthyle (préparé à l'exemple 1.C.4.) dans 400 ml d'ammoniac liquide. Ensuite, on évapore le mélange sous pression réduite et on purifie le résidu obtenu par chromatographie sur gel de silice (éluant: mélange acétate d'éthyle-éthanol-ammoniaque 80:20:0,5 v/v/v). On obtient 3,3 g de 6-hydroxy-3-[(1H-imidazol-4-yl)méthyl]-2-méthylbenzamide. Rendement: 61%.
L'amide, traité dans l'éthanol avec 1,1 équivalent d'acide chlorhydrique, forme un chlorhydrate. Rendement: 66% (après recristallisation dans l'eau). P.F.: 262,8°C.
Analyse pour $C_{12}H_{13}N_3O_2$.HCl en %:

| | | | | |
|---|---|---|---|---|
| calculé: | C 53,84 | H 5,27 | N 15,70 | $Cl^-$ 13,24 |
| trouvé: | 54,22 | 5,29 | 15,74 | 13,12 |

12. chlorhydrate de 2-hydroxy-3-[(1H-imidazol-4-yl)méthyl]-6-méthylbenzamide.

Ce composé a été préparé comme indiqué en 11. ci-dessus à partir de 2,0 g (7,68 mmoles) de 2-hydroxy-3-[(1H-imidazol-4-yl)méthyl]-6-méthylbenzoate d'éthyle (préparé à l'exemple 1.C.4.) et 12 ml d'ammoniac liquide. On chauffe à 60°C pendant 24 heures, puis on évapore le mélange sous pression réduite. L'amide brut est traité, sans purification préalable, avec 1,1 équivalent d'acide chlorhydrique dans un mélange éthanol-éther diéthylique. Le chlorhydrate est recristallisé dans le tétrahydrofuranne. Rendement: 49%. P.F.: 164,8°C (mélange de produits amorphe et cristallin).
Analyse pour $C_{12}H_{13}N_3O_2$.HCl en %:

| | | | | |
|---|---|---|---|---|
| calculé: | C 53,84 | H 5,27 | N 15,70 | $Cl^-$ 13,24 |
| trouvé: | 52,55 | 5,30 | 15,65 | 13,92 |

13. chlorhydrate-monohydrate de N,2-dihydroxy-3-[(1H-imidazol-4-yl)méthyl]-benzamide.

A un mélange de 4,1 g (25 mmoles) de sulfate d'hydroxylamine et de 25 g de glace pilée, on ajoute goutte à goutte une solution de 5 g (125 mmoles) d'hydroxyde de sodium dans 15 ml d'eau. Lorsque la température est revenue à 0°C, on ajoute 0,5 g de sulfite de sodium solide, puis 6,15 g (25 mmoles) de 2-hydroxy-3-[(1H-imidazol-4-yl)méthyl]-benzoate d'éthyle. Après dissolution complète, le milieu réactionnel est neutralisé avec 20,8

ml d'une solution aqueuse d'acide chlorhydrique 6N. On filtre le précipité blanc obtenu, on le lave à l'eau et on le purifie par chromatographie sur gel de silice (éluant: mélange dichlorométhane-méthanol-acide acétique-eau 76:20:2:2 v/v/v/v). L'acétate obtenu après évaporation des solvants est repris par 30 ml d'eau auxquels on ajoute 5 ml d'acide chlorhydrique concentré. Le chlorhydrate de N,2-dihydroxy-3-[(1H-imidazol-4-yl)méthyl]-benzamide précipite sous forme de monohydrate. On obtient 4,8 g de produit pur. Rendement: 55%. P.F.: 240°C (décomp.).
Analyse pour $C_{11}H_{11}N_3O_3.HCl.H_2O$ en %:

| | | | | |
|---|---|---|---|---|
| calculé: | C 45,92 | H 4,90 | N 14,61 | $Cl^-$ 12,32 |
| trouvé: | 46,33 | 4,63 | 14,69 | 12,57 |

Le 2-hydroxy-3-[(1H-imidazol-4-yl)méthyl]-benzoate d'éthyle, utilisé comme produit de départ, a été préparé par le procédé décrit à l'exemple 7 de la demande de brevet européen n° 269.599 au nom de la demanderesse.

14. 2-hydroxy-3-[(1H-imidazol-4-yl)méthyl]-4-méthylbenzamide.

Ce composé a été préparé comme indiqué en 2. ci-dessus à partir du bromhydrate de 2-hydroxy-3-[(1H-imidazol-4-yl)méthyl]-4-méthylbenzoate de méthyle (préparé à l'exemple 1.C.5.). Le produit de réaction est purifié par chromatographie sur gel de silice (éluant: mélange dichlorométhane-méthanol-ammoniaque 95:5:0,5 v/v/v).
Rendement: 44%. P.F.: 172-178°C (éther diéthylique).
Analyse pour $C_{12}H_{13}N_3O_2$ en %:

| | | | |
|---|---|---|---|
| calculé: | C 62,33 | H 5,62 | N 18,18 |
| trouve | 62,42 | 5,61 | 18,08 |

15. chlorhydrate de 2-hydroxy-3-[1-(1H-imidazol-4-yl)pentyl]-benzamide.

Ce composé a été préparé comme indiqué en 5. ci-dessus à partir de 2-hydroxy-3-[1-(1H-imidazol-4-yl)pentyl]-benzoate d'éthyle (préparé à l'exemple 1.A.3.). Le résidu obtenu après évaporation du solvant est dissous dans une solution d'acide chlorhydrique 2,5N dans l'éthanol et le chlorhydrate du 2-hydroxy-3-[1-(1H-imidazol-4-yl)pentyl]-benzamide précipite par addition d'éther diéthylique. Rendement: 50%.
Spectre RMN (DMSO): delta 0,83(3H,t), 1,08 à 1,34(4H,m), 3,3 à 3,5(2H,m), 4,44(1H,t), 6,82(1H,t), 7,37 à 7,40(2H,s+d), 7,82(1H,d), 8,97(1H,s).
Les composés suivants ont été préparés de la même manière.

16. 2-hydroxy-3-[(1H-imidazol-4-yl)méthyl]-N,N-diméthylbenzamide.

P.F.: 208°C.
Analyse pour $C_{13}H_{16}N_3O_2$ en %:

| | | | |
|---|---|---|---|
| calculé: | C 63,67 | H 6,12 | N 17,14 |
| trouvé: | 63,58 | 6,06 | 17,09 |

17. chlorhydrate de 2-hydroxy-N-(2-hydroxyéthyl)-3-[(1H-imidazol-4-yl)méthyl]-benzamide.

P.F.: 200,6°C.
Analyse pour $C_{13}H_{15}N_3O_3.HCl$ en %:

| | | | |
|---|---|---|---|
| calculé: | C 52,44 | H 5,38 | N 14,12 |
| trouvé: | 52,49 | 5,36 | 13,98 |

Exemple 5. Préparation des 1-(1H-imidazol-4-yl)alkyl-benzamides de formule I par réaction des acides de formule IV avec un composé azoté de formule III.

1. chlorhydrate de 3-[(1H-imidazol-4-yl)méthyl]-2-méthoxybenzamide.

On refroidit à 0°C une suspension de 3,1 g (13,4 mmoles) d'acide 3-[(1H-imidazol-4-yl)méthyl]-2-méthoxybenzoïque (préparé à l'exemple 2.B.1.) et de 4,06 g (40,2 mmoles) de triéthylamine dans 30 ml de dichlorométhane sec. On y ajoute 4,36 g (40,2 mmoles) de chloroformiate d'éthyle en solution dans 10 ml de dichlorométhane sec. Après l'addition, on agite encore pendant une demi-heure à 0°C et ensuite pendant une demi-heure à la température ambiante. On fait alors passer dans le milieu réactionnel, pendant une nuit, un courant de gaz ammoniac séché sur de l'hydroxyde de potassium. Ensuite, on chauffe le mélange à reflux pendant une demi-heure, on évapore le solvant sous pression réduite et on purifie le résidu par chromatographie sur gel de silice (éluant: mélange dichlorométhane-méthanol-ammoniaque 89:10:1 v/v/v). On obtient 2,9 g de 3-[(1H-imidazol-4-yl)méthyl]-2-méthoxybenzamide, qui cristallise dans l'acétonitrile. Rendement: 95%.
L'amide, traité avec 1,2 équivalent d'acide chlorhydrique dans l'éthanol, forme un chlorhydrate. P.F.: 160,5°C (alcool isopropylique).
Analyse pour $C_{12}H_{13}N_3O_2$.HCl en %:

| | | | |
|---|---|---|---|
| calculé: | C 53,84 | H 5,27 | N 15,73 |
| trouvé: | 53,94 | 5,30 | 15,81 |

Le composé suivant a été préparé de la même manière:

2. 3-[(1H-imidazol-4-yl)méthyl]-2-n-propoxybenzamide.

Ce composé a été préparé à partir de l'acide 3-[(1H-imidazol-4-yl)méthyl]-2-n-propoxybenzoïque (préparé à l'exemple 2.B.2.) avec un rendement de 52%. P.F.: 161°C.
Analyse pour $C_{14}H_{17}N_3O_2$ en %:

| | | | |
|---|---|---|---|
| calculé: | C 64,86 | H 6,56 | N 16,22 |
| trouvé: | 64,93 | 6,60 | 16,14 |

Exemple 6. Préparation des 1-(1H-imidazol-4-yl)alkyl-benzamides de formule I par hydrolyse des 2-hydroxy-3-[1-(1H-imidazol-4-yl)alkyl]-benzonitriles de formule V.

1. 2-hydroxy-3-[(1H-imidazol-4-yl)méthyl]-benzamide.

On agite jusqu'à dissolution complète, 13,1 g (66 mmoles) de 2-hydroxy-3-[(1H-imidazol-4-yl)méthyl]-benzonitrile (préparé à l'exemple 3.1.) dans 50 ml d'une solution aqueuse d'acide sulfurique à 80% en volume. On chauffe ensuite à 65°C pendant trois heures. Le mélange réactionnel est versé sur de la glace et neutralisé par du bicarbonate de sodium. On filtre et on extrait le filtrat plusieurs fois par de l'acétate d'éthyle. Les phases organiques sont lavées avec une solution aqueuse saturée de chlorure de sodium, séchées sur du sulfate de sodium et évaporées sous pression réduite. Le résidu est purifié par chromatographie sur gel de silice (éluant: mélange dichlorométhane-méthanol-ammoniaque 84:15:1 v/v/v). On obtient 9,8 g de 2-hydroxy-3-[(1H-imidazol-4-yl)méthyl]-benzamide, identique au produit obtenu à l'exemple 4.1. Rendement: 68%.

2. 2-hydroxy-3-[1-(1H-imidazol-4-yl)éthyl]-benzamide.

On agite à 65°C pendant trois heures une suspension de 1,07 g (5 mmoles) de 2-hydroxy-3-[1-(1H-imidazol-4-yl)éthyl]-benzonitrile (préparé à l'exemple 3.2.) dans 4 ml d'une solution aqueuse d'acide sulfurique à 80% en volume. Le mélange réactionnel est ensuite versé sur de la glace et neutralisé par du bicarbonate de sodium. On extrait plusieurs fois par de l'acétate d'éthyle. Les phases organiques sont séchées sur du sulfate de sodium et le solvant est évaporé sous pression réduite. On reprend le résidu par 50 ml d'une solution aqueuse d'acide chlorhydrique 6N et on neutralise ensuite au moyen d'une solution aqueuse d'hydroxyde de sodium 1N. Le précipité qui s'est formé est filtré, lavé à l'eau et à l'éther diéthylique et séché sous vide. On obtient 0,7 g de 2-hydroxy-3-[1-(1H-imidazol-4-yl)éthyl]-benzamide pratiquement pur. Rendement: 70%.

Le chlorhydrate du produit obtenu est identique à celui préparé à l'exemple 4.5.

3. chlorhydrate de 2-hydroxy-3-[(1H-imidazol-4-yl)méthyl]-benzamide.

On ajoute 2 g de 2-hydroxy-3-[(1H-imidazol-4-yl)méthyl]-benzonitrile (préparé à l'exemple 3.1.) et 4 ml d'eau dans 40 ml de méthanol préalablement saturé à -10°C par un courant d'acide chlorhydrique gazeux. On agite le mélange pendant 24 heures à la température ambiante. On concentre ensuite la solution sous pression réduite et on chauffe le résidu à 75°C pendant 3 heures. Ce dernier est ensuite recristallisé deux fois dans l'eau. On obtient 1,5 g de chlorhydrate de 2-hydroxy-3-[(1H-imidazol-4-yl)méthyl]-benzamide, identique au produit obtenu à l'exemple 4.1. Rendement: 59%.

## Revendications

### Revendications pour les Etats contractants suivants : AT, BE, CH, DE, FR, GB, IT, LI, LU, NL, SE

**1.** 1-(1H-imidazol-4-yl)alkyl-benzamides substitués, leurs isomères optiques et leurs mélanges racémiques répondant à la formule générale

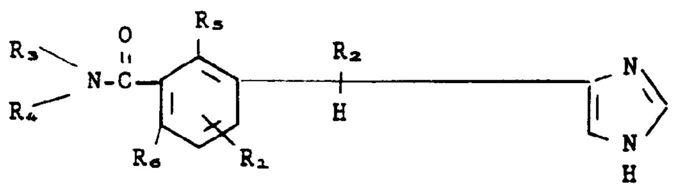

(I)

dans laquelle

$R_1$ et $R_2$, qui peuvent être identiques ou différents, représentent chacun un atome d'hydrogène ou un radical alkyle,

$R_3$ représente un atome d'hydrogène, un radical alkyle ou hydroxyalkyle, un groupe amino ou hydroxyle,

$R_4$ représente un atome d'hydrogène ou un radical alkyle, ou

$R_3$ et $R_4$ pris ensemble avec l'atome d'azote auquel ils sont attachés représentent un radical hétérocyclique choisi parmi les radicaux pyrrolidino, pipéridino et morpholino, et

$R_5$ et $R_6$, qui peuvent être identiques ou différents, représentent chacun un atome d'hydrogène, un groupe hydroxyle, un radical alkyle ou alkoxy,

au moins un des symboles $R_6$ et $R_6$ ayant une signification autre que de l'hydrogène,

tous les radicaux alkyle et alkoxy ayant de 1 à 4 atomes de carbone, ainsi que leurs sels d'addition d'acides non toxiques pharmaceutiquement acceptables.

**2.** 2-hydroxy-3-[(1H-imidazol-4-yl)méthyl]-benzamide selon la revendication 1 et ses sels d'addition d'acides non toxiques pharmaceutiquement acceptables.

**3.** 2-hydroxy-3-[1-(1H-imidazol-4-yl)éthyl]-benzamide selon la revendication 1 et ses sels d'addition d'acides non toxiques pharmaceutiquement acceptables.

**4.** 2-hydroxy-3-[(1H-imidazol-4-yl)méthyl]-N-méthylbenzamide selon la revendication 1 et ses sels d'addition d'acides non toxiques pharmaceutiquement acceptables.

**5.** 2,6-dihydroxy-3-[(1H-imidazol-4-yl)méthyl]-benzamide selon la revendication 1 et ses sels d'addition d'acides non toxiques pharmaceutiquement acceptables.

**6.** 2-hydroxy-3-[(1H-imidazol-4-yl)méthyl]-6-méthylbenzamide selon la revendication 1 et ses sels d'addition d'acides non toxiques pharmaceutiquement acceptables.

**7.** 2-hydroxy-5-[(1H-imidazol-4-yl)méthyl]benzamide selon la revendication 1 et ses sels d'addition d'acides non toxiques pharmaceutiquement acceptables.

**8.** 2-hydroxy-3-[(1H-imidazol-4-yl)méthyl]-benzohydrazide selon la revendication 1 et ses sels d'addition d'acides non toxiques pharmaceutiquement acceptables.

**9.** (+)-2-hydroxy-3-[1-(1H-imidazol-4-yl)éthyl]-benzamide selon la revendication 1 et ses sels d'addition d'acides non toxiques pharmaceutiquement acceptables.

**10.** Procédé de préparation de 1-(1H-imidazol-4-yl)alkyl-benzamides substitués et de leurs sels tels que définis dans la revendication 1, caractérisé en ce que:

a) on fait réagir un 1-(1H-imidazol-4-yl)alkyl-benzoate d'alkyle de formule

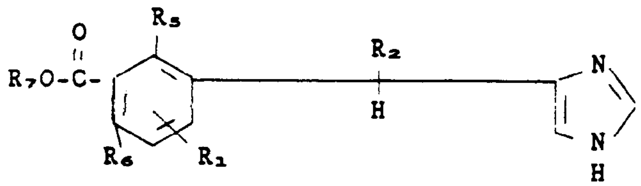

(II)

dans laquelle $R_1$, $R_2$, $R_5$ et $R_5$ ont la signification donnée à la revendication 1 et $R_7$ représente un radical alkyle ayant 1 à 4 atomes de carbone, avec un composé azoté de formule

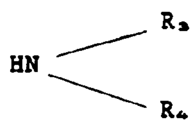

(III)

dans laquelle $R_3$ et $R_4$ ont la signification donnée à la revendication 1; ou

b) pour préparer les 1-(1H-imidazol-4-yl)alkyl-benzamides répondant à la formule générale I dans laquelle $R_5$ et $R_5$ représentent chacun de l'hydrogène, un radical alkyle ou alkoxy ayant 1 à 4 atomes de carbone, au moins un des symboles $R_5$ et $R_5$ ayant une signification autre que de l'hydrogène, on fait réagir un acide 1-(1H-imidazol-4-yl)alkyl-benzoïque de formule

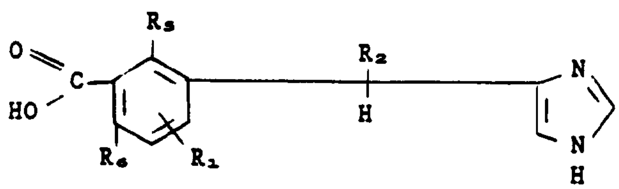

(IV)

dans laquelle $R_1$ et $R_2$ ont la signification donnée à la revendication 1 et $R_5$ et $R_5$ ont la signification donnée ci-dessus, avec un composé azoté de formule

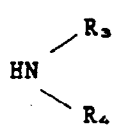

(III)

dans laquelle $R_3$ et $R_4$ ont la signification donnée à la revendication 1; ou

c) pour préparer les 1-(1H-imidazol-4-yl)alkyl-benzamides répondant à la formule générale I dans laquelle $R_1$, $R_3$ et $R_4$ représentent de l'hydrogène, $R_5$ représente un groupe hydroxyle et $R_5$ représente de l'hydrogène ou un radical alkyle ayant 1 à 4 atomes de carbone, on hydrolyse en milieu acide un 2-hydroxy-3-[1-(1H-imidazol-4-yl)alkyl]-benzonitrile de formule

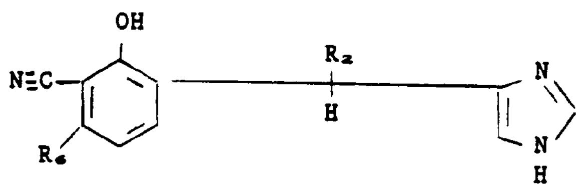

(V)

dans laquelle $R_2$ a la signification donnée à la revendication 1 et $R_6$ a la signification donnée ci-dessus; et

d) en ce qu'éventuellement, on convertit les 1-(1H-imidazol-4-yl)alkyl-benzamides de formule I ainsi obtenus en leurs sels d'addition d'acides non toxiques pharmaceutiquement acceptables.

**11.** 1-(1H-imidazol-4-yl)alkyl-benzamides selon l'une quelconque des revendications 1 à 9 ou un de leurs sels d'addition d'acides non toxiques pharmaceutiquement acceptables pour utilisation en thérapie comme agents anti-ischémiques.

**12.** 1-(1H-imidazol-4-yl)alkyl-benzamides selon l'une quelconque des revendications 1 à 9 ou un de leurs sels d'addition d'acides non toxiques pharmaceutiquement acceptables pour utilisation dans le traitement ou la prévention des pathologies associées à une élévation anormale des taux de catécholamines.

**13.** Compositions thérapeutiques renfermant une quantité thérapeutiquement efficace d'un 1-(1H-imidazol-4-yl)alkyl-benzamide selon l'une quelconque des revendications 1 à 9 ou d'un de ses sels d'addition d'acides non toxiques pharmaceutiquement acceptables, en association avec des excipients pharmaceutiques solides ou liquides.

**Revendication pour les Etats contractants suivants: ES, GR**

**1.** Procédé de préparation de 1-(1H-imidazol-4-yl)alkyl-benzamides substitués, leurs isomères optiques et leurs mélanges racémiques répondant à la formule générale

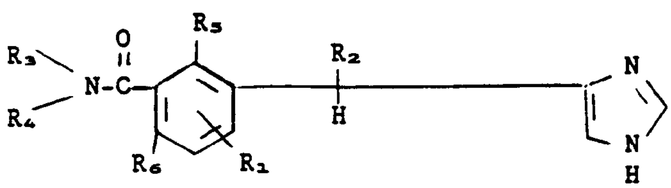

(I)

dans laquelle

$R_1$ et $R_2$, qui peuvent être identiques ou différents, représentent chacun un atome d'hydrogène ou un radical alkyle,

$R_3$ représente un atome d'hydrogène, un radical alkyle ou hydroxyalkyle, un groupe amino ou hydroxyle,

$R_4$ représente un atome d'hydrogène ou un radical alkyle, ou

$R_3$ et $R_4$ pris ensemble avec l'atome d'azote auquel ils sont attachés représentent un radical hétérocyclique choisi parmi les radicaux pyrrolidino, pipéridino et morpholino, et

$R_5$ et $R_6$, qui peuvent être identiques ou différents, représentent chacun un atome d'hydrogène, un groupe hydroxyle, un radical alkyle ou alkoxy,

au moins un des symboles $R_6$ et $R_6$ ayant une signification autre que de l'hydrogène,

tous les radicaux alkyle et alkoxy ayant de 1 à 4 atomes de carbone, ainsi que leurs sels d'addition d'acides non toxiques pharmaceutiquement acceptables, caractérisé en ce que:

a) on fait réagir un 1-(1H-imidazol-4-yl)alkyl-benzoate d'alkyle de formule

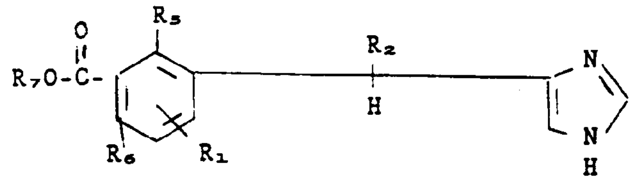

(II)

dans laquelle $R_1$, $R_2$, $R_5$ et $R_5$ ont la signification donnée ci-dessus et $R_7$ représente un radical alkyle ayant 1 à 4 atomes de carbone, avec un composé azoté de formule

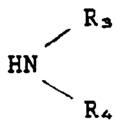

(III)

dans laquelle $R_3$ et $R_4$ ont la signification donnée ci-dessus; ou

b) pour préparer les 1-(1H-imidazol-4-yl)alkyl-benzamides répondant à la formule générale I dans laquelle $R_5$ et $R_5$ représentent chacun de l'hydrogène, un radical alkyle ou alkoxy ayant 1 à 4 atomes de carbone, au moins un des symboles $R_5$ et $R_5$ ayant une signification autre que de l'hydrogène, on fait réagir un acide 1-(1H-imidazol-4-yl)alkyl-benzoïque de formule

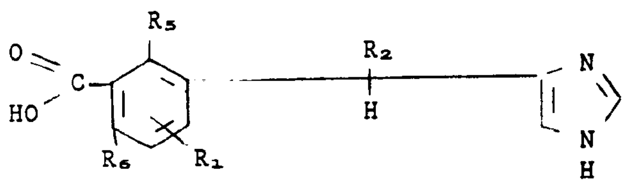

(IV)

dans laquelle $R_1$, $R_2$, $R_5$ et $R_6$ ont la signification donnée ci-dessus, avec un composé azoté de formule

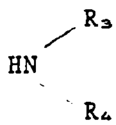

(III)

dans laquelle $R_3$ et $R_4$ ont la signification donnée ci-dessus, ou

c) pour préparer les 1-(1H-imidazol-4-yl)alkyl-benzamides répondant à la formule générale I dans laquelle $R_1$, $R_3$ et $R_4$ représentent de l'hydrogène, $R_5$ représente un groupe hydroxyle et $R_5$ représente de l'hydrogène ou un radical alkyle ayant 1 à 4 atomes de carbone, on hydrolyse en milieu acide un 2-hydroxy-3-[1-(1H-imidazol-4-yl)alkyl]-benzonitrile de formule

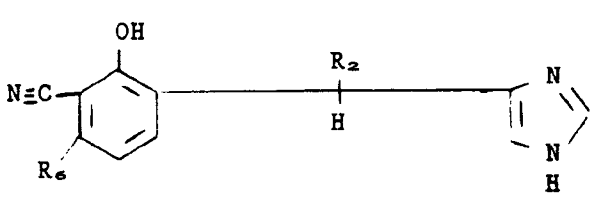

(V)

dans laquelle $R_2$ et $R_6$ ont la signification donnée ci-dessus; et

d) en ce qu'éventuellement, on convertit les 1-(1H-imidazol-4-yl)alkyl-benzamides de formule I ainsi obtenus en leurs sels d'addition d'acides non toxiques pharmaceutiquement acceptables.

## Patentansprüche

### Patentansprüche für folgende Vertragsstaaten: AT, BE, CH, DE, FR, GB, IT, LI, LU, NL, SE

1. Substituierte 1-(1H-imidazol-4-yl)alkyl-benzamide, deren optische Isomeren und deren racemische Mischungen, der allgemeinen Formel

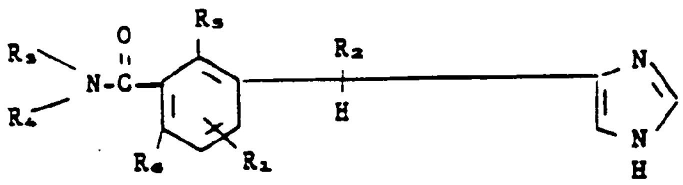

(I)

in der

$R_1$ und $R_2$, die gleich oder unterschiedlich sein können, jeweils ein Wasserstoffatom oder einen Alkylrest darstellen,

$R_3$ ein Wasserstoffatom, einen Alkyl- oder Hydroxyalkylrest, eine Amino- oder-Hydroxylgruppe darstellt,

$R_4$ ein Wasserstoffatom oder einen Alkylrest oder

$R_3$ und $R_4$ zusammen mit dem Stickstoffatom, an das sie gebunden sind, einen heterozyklischen Rest, ausgewählt unter den Pyrrolidino-, Piperidino- und Morpholinoresten, darstellen, und

$R_5$ und $R_6$, die gleich oder unterschiedlich sein können, jeweils ein Wasserstoffatom, eine Hydroxylgruppe, einen Alkyl- oder Alkoxyrest darstellen,

wobei wenigstens eines der Symbole $R_6$ und $R_6$ eine andere Bedeutung als Wasserstoff aufweist,

wobei alle Alkyl- und Alkoxyreste 1 bis 4 Kohlenstoffatome aufweisen,

sowie deren Additions-Salze nicht-toxischer, pharmazeutisch annehmbarer Säuren.

2. 2-Hydroxy-3-[(1H-imidazol-4-yl)methyl]-benzamid nach Anspruch 1 und dessen Additions-Salze nicht-toxischer, pharmazeutisch annehmbarer Saüren.

3. 2-Hydroxy-3-[1-(1H-imidazol-4-yl)ethyl]-benzamid nach Anspruch 1 und dessen Additions-Salze nicht-toxischer, pharmazeutisch annehmbarer Saüren.

4. 2-Hydroxy-3-[(1H-imidazol-4-yl)methyl]-N-methylbenzamid nach Anspruch 1 und dessen Additions-Salze nicht-toxischer, pharmazeutisch annehmbarer Saüren.

5. 2,6-Dihydroxy-3-[(1H-imidazol-4-yl)methyl]-benzamid nach Anspruch 1 und dessen Additions-Salze nicht-toxischer, pharmazeutisch annehmbarer Saüren.

6. 2-Hydroxy-3-[(1H-imidazol-4-yl)methyl]-6-methylbenzamid nach Anspruch 1 und dessen Additions-Salze nicht-toxischer, pharmazeutisch annehmbarer Saüren.

7. 2-Hydroxy-5-[(1H-imidazol-4-yl)methyl]-benzamid nach Anspruch 1 und dessen Additions-Salze nicht-toxischer, pharmazeutisch annehmbarer Saüren.

8. 2-Hydroxy-3-[(1H-imidazol-4-yl)methyl]-benzohydrazid nach Anspruch 1 und dessen Additions-Salze nicht-toxischer, pharmazeutisch annehmbarer Saüren.

9. (+)-2-Hydroxy-3-[1-(1H-imidazol-4-yl)ethyl]-benzamid nach Anspruch 1 und dessen Additions-Salze nicht-toxischer, pharmazeutisch annehmbarer Saüren.

10. Verfahren zur Herstellung von substituierten 1-(1H-Imidazol-4-yl)alkyl-benzamiden und deren Salzen, wie in Anspruch 1 definiert, dadurch gekennzeichnet, daß man

a) ein Alkyl-1-(1H-imidazol-4-yl)alkyl-benzoat der Formel

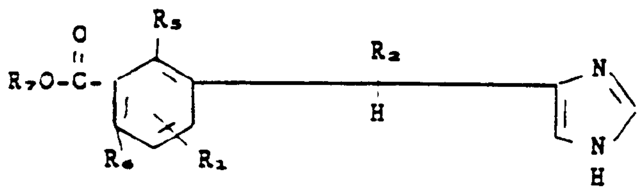

(II)

in der $R_1$, $R_2$, $R_5$ und $R_6$ die in Anspruch 1 gegebene Bedeutung aufweisen und $R_7$ einen Alkylrest mit 1 bis 4 Kohlenstoffatomen darstellt, mit einer stickstoffhaltigen Verbindung der Formel

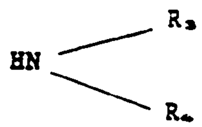

(III)

in der $R_3$ und $R_4$ die in Anspruch 1 gegebene Bedeutung aufweisen, reagieren läßt; oder

b) zur Herstellung der 1-(1H-Imidazol-4-yl)alkyl-benzamide der allgemeinen Formel I in der $R_5$ und $R_5$ jeweils Wasserstoff, einen Alkyl- oder Alkoxyrest mit 1 bis 4 Kohlenstoffatomen darstellen, wobei wenigstens eines der Symbole $R_5$ und $R_6$ eine andere Bedeutung als Wasserstoff aufweist, eine 1-(1H-Imidazol-4-yl)alkyl-benzoesäure der Formel

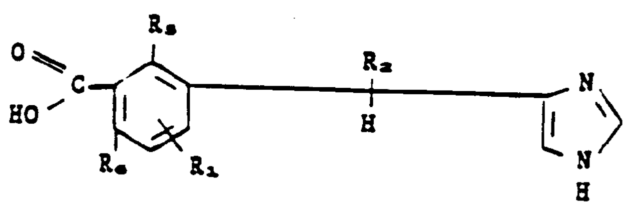

(IV)

in der $R_1$ und $R_2$ die Anspruch 1 gegebene Bedeutung aufweisen und $R_5$ und $R_6$ die obengennante Bedeutung aufweisen, mit einer stickstoffhaltigen Verbindung der Formel

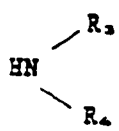

(III)

in der $R_3$ und $R_4$ die in Anspruch 1 gegebene Bedeutung aufweisen, reagieren läßt; oder

c) zur Herstellung der 1-(1H-Imidazol-4-yl)alkyl-benzamide der allgemeinen Formel I, in der $R_1$, $R_3$ und $R_4$ Wasserstoff darstellen, $R_5$ eine Hydroxylgruppe darstellt und $R_5$ Wasserstoff oder einen Alkylrest mit 1 bis 4 Kohlenstoffatomen darstellt, in sauren Medium ein 2-Hydroxy-3-[1-(1H-imidazol-4-yl)alkyl]-benzonitril der Formel

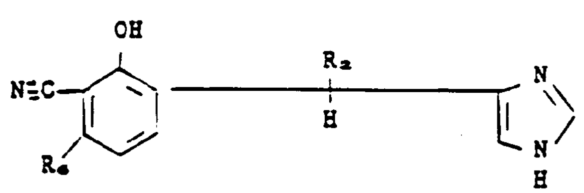

(V)

in der $R_2$ die in Anspruch 1 gegebene Bedeutung und $R_6$ die oben genannte Bedeutung aufweist, hydrolysiert; und

d) gegebenenfalls, die so erhaltenen 1-(1H-Imidazol-4-yl)alkyl-benzamide der Formel I in ihre Additions-Salze nicht-toxischer, pharmazeutisch annehmbarer Säuren umwandelt.

11. 1-(1H-Imidazol-4-yl)alkyl-benzamide nach einem der Ansprüche 1 bis 9 oder eines ihrer Additions-Salze nicht-toxischer, pharmazeutisch annehmbarer Säuren zur Verwendung bei der Therapie als anti-ischämische Mittel.

12. 1-(1H-Imidazol-4-yl)alkyl-benzamide nach einem der Ansprüche 1 bis 9 oder eines ihrer Additions-Salze nicht-toxischer, pharmazeutisch annehmbarer Säuren zur Verwendung bei der Behandlung oder Verhinderung von mit einer anormalen Erhöhung der Katecholaminwerte verbundenen Pathologien.

13. Heilmittel enthaltend eine therapeutisch wirksame Menge eines 1-(1H-Imidazol-4-yl)alkyl-benzamids nach einem der Ansprüche 1 bis 9 oder eines seiner Additions-Salze nicht-toxischer, pharmazeutisch annehmbarer Säuren in Verbindung mit festen oder flüssigen pharmazeutischen Hilfstoffen.

**Patentanspruch für folgende Vertragsstaaten: ES, GR**

1. Verfahren zur Herstellung von substituierten 1-(1H-Imidazol-4-yl)alkyl-benzamiden, deren optischen Isomeren und deren racemischen Mischungen, der allgemeinen Formel

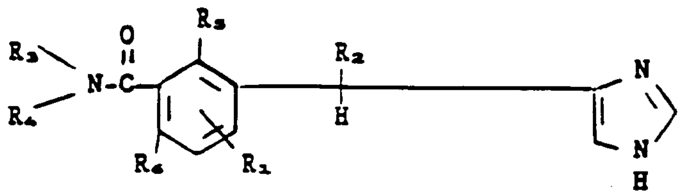

(I)

in der

$R_1$ und $R_2$, die gleich oder unterschiedlich sein können, jeweils ein Wasserstoffatom oder einen Alkylrest darstellen,

$R_3$ ein Wasserstoffatom, einen Alkyl- oder Hydroxyalkylrest, eine Amino- oder Hydroxylgruppe darstellt,

$R_4$ ein Wasserstoffatom oder einen Alkylrest oder

$R_3$ und $R_4$ zusammen mit dem Stickstoffatom, an das sie gebunden sind, einen heterozyklischen Rest, ausgewählt unter den Pyrrolidino-, Piperidino- und Morpholinoresten, darstellen, und

$R_5$ und $R_6$, die gleich oder unterschiedlich sein können, jeweils ein Wasserstoffatom, eine Hydroxylgruppe, einen Alkyl- oder Alkoxyrest darstellen,

wobei wenigstens eines der Symbole $R_6$ und $R_6$ eine andere Bedeutung als Wasserstoff aufweist, wobei alle Alkyl- und Alkoxyreste 1 bis 4 Kohlenstoffatome aufweisen, sowie deren Additions-Salze nicht-toxischer, pharmazeutisch annehmbarer Säuren, dadurch gekennzeichnet, daß man

a) ein Alkyl-1-(1H-imidazol-4-yl)alkyl-benzoat der Formel

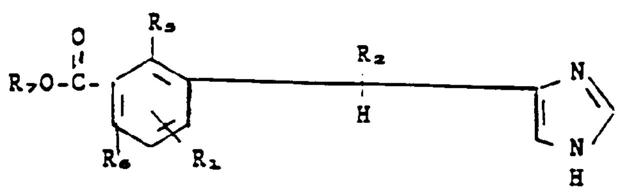

(II)

in der $R_1$, $R_2$, $R_5$ und $R_6$ die obengenannte Bedeutung aufweisen und $R_7$ einen Alkylrest mit 1 bis 4 Kohlenstoffatomen darstellt, mit einer stickstoffhaltigen Verbindung der Formel

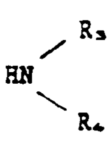

(III)

in der R und $R_4$ die obengenannte Bedeutung aufweisen, reagieren läßt; oder

b) zur Herstellung der 1-(1H-Imidazol-4-yl)alkyl-benzamide der allgemeinen Formel I, in der $R_6$ und $R_6$

jeweils Wasserstoff, einen Alkyl- oder Alkoxyrest mit 1 bis 4 Kohlenstoffatomen darstellen, wobei wenigstens eines der Symbole $R_5$ und $R_6$ eine andere Bedeutung als Wasserstoff aufweist, eine 1-(1H-Imidazol-4-yl)alkyl-benzoesäure der Formel

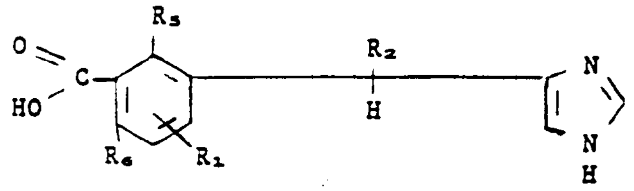

(IV)

in der $R_1$, $R_2$, $R_5$ und $R_6$ die obengenannte Bedeutung aufweisen, mit einer stickstoffhaltigen Verbindung der Formel

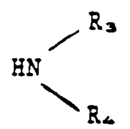

(III)

in der $R_3$ und $R_4$ die obengenannte Bedeutung aufweisen, reagieren läßt; oder

c) zur Hertellung der 1-(1H-Imidazol-4-yl)alkyl-benzamide der allgemeinen Formel I, in der $R_1$, $R_3$ und $R_4$ Wasserstoff darstellen, $R_5$ eine Hydroxylgruppe darstellt und $R_5$ Wasserstoff oder einen Alkylrest mit 1 bis 4 Kohlenstoffatomen darstellt, in saurem Medium ein 2-Hydroxy-3-[1-(1H-Imidazol-4-yl)alkyl]-benzonitril der Formel

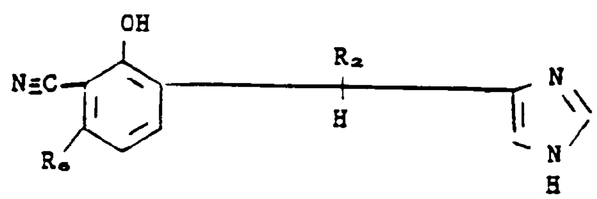

(V)

in der $R_2$ und $R_5$ die obengenannte Bedeutung aufweisen, hydrolysiert; und

d) gegebenenfalls, die so erhaltenen 1-(1H-Imidazol-4-yl)alkyl-benzamide der Formel I in ihre Additions-Salze nicht-toxischer, pharmazeutisch annehmbarer Säuren umwandelt.

## Claims

### Claims for the following Contracting States: AT, BE, CH, DE, FR, GB, IT, LI, LU, NL, SE

**1.** Substituted 1-(1H-imidazol-4-yl)alkyl-benzamides, their optical isomers and their racemic mixtures, having the general formula

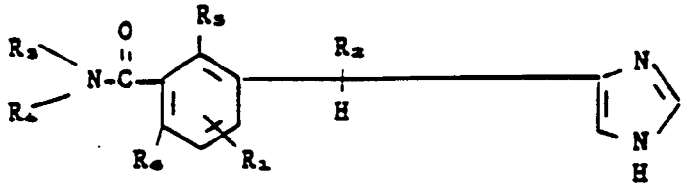

(I)

wherein

$R_1$ and $R_2$, which may be the same or different, each represent a hydrogen atom or an alkyl radical,
$R_3$ represents a hydrogen atom, an alkyl or hydroxyalkyl radical, an amino or hydroxyl group,
$R_4$ represents a hydrogen atom or an alkyl radical, or

$R_3$ and $R_4$, taken together with the nitrogen atom to which they are attached, represent a heterocyclic radical selected from the pyrrolidino, piperidino and morpholino radicals, and

$R_5$ and $R_6$, which may be the same or different, each represent a hydrogen atom, a hydroxyl group, an alkyl or alkoxy radical,

at least one of the symbols $R_5$ and $R_6$ having a meaning other than a hydrogen atom, all the alkyl and alkoxy radicals having 1 to 4 carbon atoms,

as well as their non-toxic pharmaceutically acceptable acid addition salts.

2. 2-hydroxy-3-[(1H-imidazol-4-yl)methyl]-benzamide according to claim 1 and its non-toxic, pharmaceutically acceptable acid addition salts.

3. 2-hydroxy-3-[1-(1H-imidazol-4-yl)ethyl]-benzamide according to claim 1 and its non-toxic, pharmaceutically acceptable acid addition salts.

4. 2-hydroxy-3-[(1H-imidazol-4-yl)methyl]-N-methylbenzamide according to claim 1 and its non-toxic, pharmaceutically acceptable acid addition salts.

5. 2,6-dihydroxy-3-[(1H-imidazol-4-yl)methyl]-benzamide according to claim 1 and its non-toxic, pharmaceutically acceptable acid addition salts.

6. 2-hydroxy-3-[(1H-imidazol-4-yl)methyl]-6-methylbenzamide according to claim 1 and its non-toxic, pharmaceutically acceptable acid addition salts.

7. 2-hydroxy-5-[(1H-imidazol-4-yl)methyl]-benzamide according to claim 1 and its non-toxic, pharmaceutically acceptable acid addition salts.

8. 2-hydroxy-3-[(1H-imidazol-4-yl)methyl]-benzohydrazide according to claim 1 and its non-toxic, pharmaceutically acceptable acid addition salts.

9. (+)-2-hydroxy-3-[1-(1H-imidazol-4-yl)ethyl]-benzamide according to claim 1 and its non-toxic, pharmaceutically acceptable acid addition salts.

10. Process for the preparation of substituted 1-(1H-imidazol-4-yl)alkyl-benzamides and their salts as defined in claim 1, characterised in that:

a) an alkyl 1-(1H-imidazol-4-yl)alkyl-benzoate of the formula

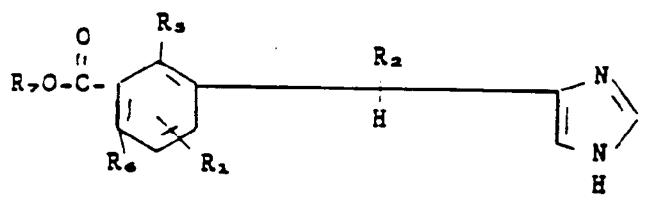

(II)

wherein $R_1$, $R_2$, $R_6$ and $R_6$ have the meaning given in claim 1 and $R_7$ represents an alkyl radical having 1 to 4 carbon atoms, is reacted with a nitrogen compound of the formula

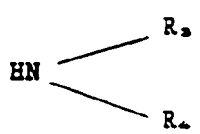

(III)

wherein $R_3$ and $R_4$ have the meaning given in claim 1; or

b) in order to prepare the 1-(1H-imidazol-4-yl)alkyl-benzamides having the general formula I in which $R_5$ and $R_6$ each represent a hydrogen atom, an alkyl or alkoxy radical having 1 to 4 carbon atoms, at least one of the symbols $R_5$ and $R_6$ having a meaning other than a hydrogen atom, a 1-(1H-imidazol-4-yl)alkyl-benzoic acid of the formula

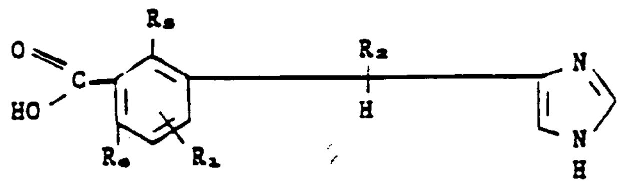

(IV)

wherein $R_1$ and $R_2$ have the meaning given in claim 1 and $R_5$ and $R_6$ have the meaning given above, is reacted with a nitrogen compound of the formula

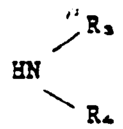

(III)

wherein $R_3$ and $R_4$ have the meaning given in claim 1; or

c) in order to prepare the 1-(1H-imidazol-4-yl)alkyl-benzamides having the general formula I, in which $R_1$, $R_3$ and $R_4$ are hydrogen atoms, $R_5$ represents a hydroxyl group and $R_6$ represents a hydrogen atom or an alkyl radical having 1 to 4 carbon atoms, a 2-hydroxy-3-[1-(1H-imidazol-4-yl)alkyl]-benzonitrile of the formula

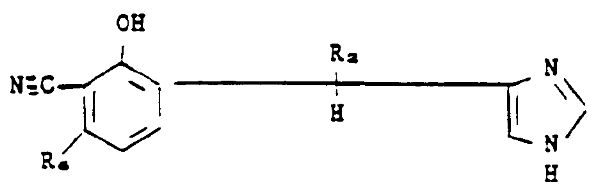

(V)

wherein $R_2$ has the meaning given in claim 1 and $R_6$ has the meaning given above, is hydrolysed in an acid medium; and

d) optionally, the 1-(1H-imidazol-4-yl)alkyl-benzamides of the formula I thus obtained are converted into their non-toxic pharmaceutically acceptable acid addition salts.

**11.** 1-(1H-imidazol-4-yl)alkyl-benzamides according to any of claims 1 to 9 or one of their non-toxic pharmaceutically acceptable acid addition salts for use in therapy as anti-ischemic agents.

**12.** 1-(1H-imidazol-4-yl)alkyl-benzamides according to any of claims 1 to 9 or one of their non-toxic pharmaceutically acceptable acid addition salts for use in the treatment or the prevention of disorders associated with an abnormal increase of the catecholamine levels.

**13.** Therapeutic compositions containing a therapeutically effective amount of a 1-(1H-imidazol-4-yl)alkyl-benzamide according to any of claims 1 to 9 or one of its non-toxic pharmaceutically acceptable acid addition salts, in association with solid or liquid pharmaceutical excipients.

**Claims for the following Contracting States: ES, GR**

**1.** Process for the preparation of substituted 1-(1H-imidazol-4-yl)alkyl-benzamides, their optical isomers and their racemic mixtures having the general formula

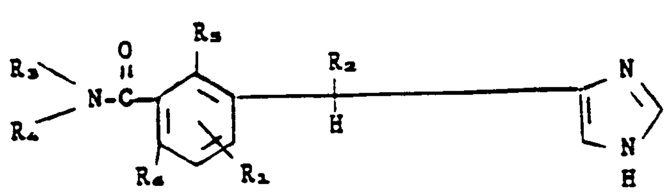

(I)

wherein

$R_1$ and $R_2$, which may be the same or different, each represent a hydrogen atom or an alkyl radical,

$R_3$ represents a hydrogen atom, an alkyl or hydroxyalkyl radical, an amino or hydroxyl group,

$R_4$ represents a hydrogen atom or an alkyl radical, or

$R_3$ and $R_4$, taken together with the nitrogen atom to which they are attached, represent a heterocyclic radical selected from the pyrrolidino, piperidino and morpholino radicals, and

$R_5$ and $R_6$, which may be the same or different, represent a hydrogen atom, a hydroxyl group, an alkyl or alkoxy radical, at least one of the symbols $R_5$ and $R_5$ having a meaning other than a hydrogen atom, all the alkyl and alkoxy radicals having 1 to 4 carbon atoms,

as well as their non-toxic pharmaceutically acceptable acid addition salts, characterised in that:

a) an alkyl 1-(1H-imidazol-4-yl)alkyl-benzoate of the formula

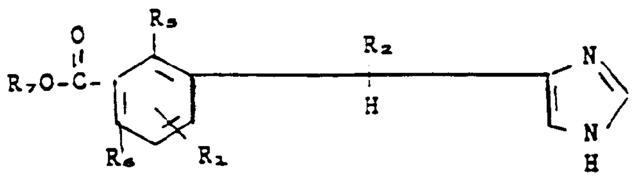

(II)

wherein $R_1$, $R_2$, $R_5$ and $R_5$ have the meaning given above and $R_7$ represents an alkyl radical having 1 to 4 carbon atoms, is reacted with a nitrogen compound of the formula

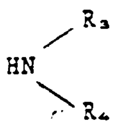

(III)

wherein $R_3$ and $R_4$ have the meaning given above; or

b) in order to prepare the 1-(1H-imidazol-4-yl)alkyl-benzamides having the general formula I, in which $R_5$ and $R_6$ each represent a hydrogen atom, an alkyl or alkoxy radical having 1 to 4 carbon atoms, at least one of the symbols $R_5$ and $R_5$ having a meaning other than a hydrogen atom, a 1-(1H-imidazol-4-yl)alkyl-benzoic acid of the formula

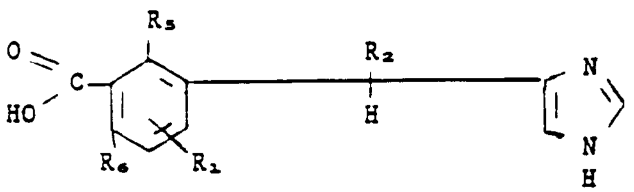

(IV)

wherein $R_1$, $R_2$, $R_5$ and $R_6$ have the meaning given above, is reacted with a nitrogen compound of the formula

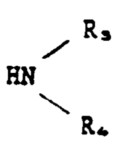

(III)

wherein $R_3$ and $R_4$ have the meaning given above; or

c) in order to prepare the 1-(1H-imidazol-4-yl)alkyl-benzamides having the general formula I, in which $R_1$, $R_3$ and $R_4$ are hydrogen atoms, $R_5$ represents a hydroxyl group and $R_6$ represents a hydrogen atom or an alkyl radical having 1 to 4 carbon atoms, a 2-hydroxy-3-[1-(1H-imidazol-4-yl)alkyl]-benzonitrile of the formula

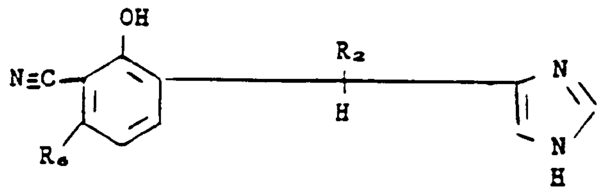

(V)

wherein $R_2$ and $R_6$ have the meaning given above, is hydrolysed in an acid medium; and
d) optionally, the 1-(1H-imidazol-4-yl)alkyl-benzamides of the formula I thus obtained are converted into their non-toxic pharmaceutically acceptable acid addition salts.